# EUROPEAN PATENT SPECIFICATION

(11) **EP 3 871 526 B1**
(45) Date of publication and mention of the grant of the patent: **01.11.2023**
(21) Application number: 18937655.1
(22) Date of filing: 19.12.2018
(51) Int. Cl.: A24F 40/42, A24F 40/10, A61M 11/04, A61M 15/06, A24F 40/40

(54) **ATOMIZATION UNIT AND NON-COMBUSTION-TYPE INHALER**
VERNEBLER UND VERBRENNUNGSFREIER INHALATOR
UNITÉ D'ATOMISATION ET INHALATEUR DE TYPE SANS COMBUSTION

(30) Priority: 26.10.2018 CN 201811255598
(43) Date of publication of application: 01.09.2021
(73) Proprietor: Japan Tobacco Inc., Tokyo 105-6927 (JP)
(72) Inventor: YAMADA, Manabu, Tokyo 130-8603 (JP); MATSUDA, Kentaro, Tokyo 130-8603 (JP); ODA, Takashi, Tokyo 130-8603 (JP); SHEN, Pifa, Shenzhen, Guangdong 518102 (CN)
(74) Representative: Hoffmann Eitle
(86) International application number: PCT/JP2018/046757
(87) International publication number: WO 2020/084791

(56) References cited:
- EP-A1- 3 193 643
- WO-A1-2017/187545
- WO-A1-2018/135635
- CN-A- 103 584 287
- CN-A- 106 235 419
- CN-U- 203 424 300
- CN-U- 203 424 300
- JP-A- 2010 270 838
- US-A1- 2013 263 869
- US-A1- 2015 327 597
- US-A1- 2015 327 597
- US-A1- 2016 192 705
- US-A1- 2016 192 710
- US-A1- 2016 324 219
- US-A1- 2016 366 938

## Description

### [Technical Field]

The present invention relates to an atomization unit and a non-combustion suction device.

### [Background Art]

Conventionally, a non-combustion suction device (hereinafter simply referred to as a suction device) configured to aspirate steam (for example, an aerosol) atomized by heating has been known. There is a device having an atomization unit in which atomizable liquid (for example, an aerosol source) is stored and a main body unit in which a storage battery is mounted as this kind of suction device.

For example, as an atomization unit, there is a configuration having a tank (protective sleeve) formed in a substantial cylindrical shape and configured to store liquid, and a holder (suction nozzle cap, battery cartridge connector) configured to block two axial ends of the tank, wherein a cotton (oil-containing cotton) to which the liquid is absorbed and a heater (heat generation member) configured to vaporize the liquid absorbed by the cotton.

If the holder is easy to be attached to or detached from the tank, for example, it is possible to refill the liquid or exchange the cotton and the heater against an intention of the manufacturer. If such situations occur, reliability of the product will be impaired. Accordingly, various technologies for making it impossible to attach the holder to the tank or detach the holder from the tank are disclosed.

For example, a technology is disclosed to form a groove portion over the whole circumference in a connection portion of the holder and the tank, form a plurality of concave portions at intervals in an external circumferential portion in a cross section in an orthogonal direction with respect to an axial direction in the groove portion, and form a protrusion portion between every two adjacent concave portions.

EP 3 193 643 A1 concerns a device for storing and vaporizing liquid media and discloses the features of the preamble of claim 1.

CN 203 424 300 U concerns an electronic cigarette. The electronic cigarette comprises an outer sleeve and a lamp cap which is installed at one end of the outer sleeve. A buckling structure is arranged on the lamp cap. The lamp cap is buckled with the outer sleeve through the buckling structure.

US 2015/327597 A1 relates to an atomizer, power supply, and electronic cigarette having the same.

US 2013/263869 A1 concerns an electronic cigarette.

### [Citation List]

### [Patent Document]

[Patent Document 1] Japanese Patent (Granted) Publication No. 6189522

### [Summary of Invention]

### [Technical Problem]

However, according to the prior art described above, since the holder is formed in the groove portion, a thickness of the position where the groove portion is formed becomes thin. Accordingly, mechanical strength at the position where the groove is formed becomes weak such that there is possibility for the holder to be simply broken.

An object of the present invention is to provide an atomization unit and a non-combustion suction device that are difficult to be intentionally disassembled and to secure efficient mechanical strength.

### [Solution to Problem]

In order to achieve the above-described object, an atomization unit according the present invention, which is defined in claim 1, has a tank formed in a bottomed cylindrical shape in which liquid is stored; and a holder fitted to an opening portion of the tank, wherein in a state in which the tank and the holder are fitted with each other, at least an engagement piece being elastically deformable towards inwardly in a radial direction is disposed in either of the tank or the holder to protrude from one of the tank and the holder positioned inwardly in the radial direction toward the other one of the tank and the holder, and an engagement portion being engageable with the engagement piece is disposed in the other one of the tank and the holder positioned outwardly in the radial direction.

According to the above, when the tank and the holder are fitted with each other, the engagement pieces may be covered by the tank or the holder from the external side in the radial direction. Accordingly, after the tank and the holder are fitted with each other, it is impossible to touch the engagement piece. In other words, after the tank and the holder are fitted with each other, it is impossible to intentionally make the engagement piece to be elastically deformed. Accordingly, it is possible to make it difficult to intentionally disassemble the tank and the holder.

There is no necessity to form the groove portion over the whole circumference of the tank and the holder as in the past to make it difficult to disassemble the tank and the holder. Accordingly, it is possible to secure efficient mechanical strength of the tank and the holder.

Further, in the atomization unit according to the present invention, an elastic applying member is formed in an internal surface at an internal side in the radial direction of the engagement piece so as to apply an elastic force outwardly in the radial direction to the engagement piece in response to an elastic deformation of the engagement piece inwardly in the radial direction.

According to the above, it is possible to cause it difficult for the engagement piece to be elastically deformed towards inwardly in the radial direction by the elastic applying member. Accordingly, it is possible to cause the engagement of the engagement portion and the engagement piece that are once engaged with each other more difficult to be released.

Still further, in the atomization unit according to the present invention, the holder is fitted to an internal circumferential surface of the tank, and the elastic applying member has a fitting portion to be fitted to an internal circumferential surface of the holder.

A length of the tank in the axial direction is longer than a length of the holder in the axial direction due to a portion storing the liquid. Accordingly, it is possible to cause the assembling of the tank and the holder to be easy by forming the engagement pieces in the holder. It is possible to cause the elastic force towards outwardly in the radial direction to be easily applied to the engagement pieces by the elastic applying member by forming the fitting portion fitted to the internal circumferential surface of the holder in the elastic applying member.

The engagement piece may have an engagement claw protruding from a tip end at the other one side towards outside in the radial direction, and the engagement portion may be an opening in which the engagement claw is insertable.

According to the above, it is possible to definitely engage the engagement piece and the engagement portion with each other using a simple structure by inserting the engagement claw into the opening. It is possible to make it difficult to cause the engagement piece to be attached to or detached from the engagement portion that are once engaged with each other.

The engagement claw may be formed to have a cross-sectional surface in a substantial triangle shape corresponding to a plane along an axial direction and a radial direction of the tank and the holder, the engagement claw may have an inclined surface which is inclined toward a base end side of the engagement piece as towards outside in the radial direction from the tip end, and a flat surface at the base end side may be orthogonal to the axial direction.

According to the above, at the time of inserting the engagement claw into the engagement portion with the opening, it is possible to cause the engagement claw to smoothly come in contact with the tank or the holder by utilizing the inclined surface. Accordingly, it is possible to smoothly insert the engagement claw into the engagement portion.

The surface at the base end side of the engagement claw is orthogonal such that once the engagement claw is inserted into the engagement portion, even if the tank or the holder is about to be pulled in the axial direction, the force for pulling is difficult to be dispersed in the radial direction. In other words, it is difficult to cause the engagement piece to be elastically deformed. Accordingly, it is possible to cause the engagement of the engagement portion and the engagement piece difficult to be released.

Two of the engagement pieces may be included, two of the engagement pieces may be oppositely disposed with a radially central portion of the tank and the holder as a center, and two of the engagement portions may be disposed corresponding to the two of the engagement pieces.

In this manner, the tank and the holder are oppositely disposed with the radially central portion as a center, when viewing the whole holder, it is difficult for a force inwardly in the radial direction that is applied to the two engagement pieces to be biased. Accordingly, the force applied for making the engagement pieces to be elastically deformed is balanced and it is easy for the engagement pieces to be engaged with the engagement portions. When the tank or the holder is inclined so as to release the either engagement of the two engagement pieces, the force outwardly in the radial direction applies on the other engagement piece. Accordingly, it is possible to cause the engagement portion and the engagement pieces that are once engaged with each other more difficult to be disassembled.

A guide concave portion configured to receive the engagement piece to guide a fitting of the tank and the holder may be formed in either of the tank or the holder.

According to the above, it is possible to perform position alignment of the tank and the holder utilizing the guide concave portion and make the fitting of the tank and the holder to be easy.

A non-combustion suction device according to the present invention is defined in claim 6 and has the atomization unit according to above; a partition plate with liquid absorbency and configured to partition the inside of the tank into a liquid storage room positioned at a bottom portion side of the tank and an opening room positioned at the opening portion side of the tank; a wick with the liquid absorbency and connected to the partition plate; and a heater having electrical heating wire for heating the wick, wherein the holder, the wick, and the heater are disposed at the opening room side.

The non-combustion suction device may further have a container retention cylinder configured to accommodate the atomization unit; a power portion connected to an end portion of the container retention cylinder at the opposite side of the bottom portion of the tank; and the holder in a bottomed cylindrical shape, wherein a bottom portion of the holder is disposed to face the opposite side of the bottom portion of the tank, and an electrode is formed in the bottom portion of the holder to be connected to the electrical heating wire and be able to come in contact with a pin electrode of the power portion.

According to the above, it is possible to provide a non-combustion suction device that is difficult to be intentionally disassembled while securing efficient mechanical strength.

### [Advantageous Effects of Invention]

According to an aspect of the present invention, it is possible to cause the tank and holder difficult to be intentionally disassembled. There is no necessity to form the groove portion over the whole circumference of the tank and the holder as in the past to make it difficult to disassemble the tank and the holder. Accordingly, it is possible to secure efficient mechanical strength of the tank and the holder.

### [Brief Description of Drawings]

Fig. 1 is a perspective view showing a non-combustion suction device according to an embodiment.
Fig. 2 is an exploded perspective view showing the non-combustion suction device according to the embodiment.
Fig. 3 is a cross-sectional view corresponding to line III-III in Fig. 1.
Fig. 4 is an exploded perspective view showing a power unit according to the embodiment.
Fig. 5 is a cross-sectional view corresponding to line V-V in Fig. 1.
Fig. 6 is a perspective view showing the power unit according to the embodiment.
Fig. 7 is a plan view showing the power unit according to the embodiment viewed from a retaining unit side in an axial direction.
Fig. 8 is an exploded perspective view showing the retaining unit according to the embodiment.
Fig. 9 is a perspective view showing a connection structure of a first connection member and a second connection member according to the embodiment.
Fig. 10 is a plan view showing the retaining unit and a cartridge according to the present embodiment viewed from the power unit side in the axial direction.
Fig. 11 is a cross-sectional view corresponding to line XI-XI in Fig. 1.
Fig. 12 is an exploded perspective view showing a mouthpiece corresponding to line XII-XII in Fig. 1.
Fig. 13 is a cross-sectional view showing the cartridge according to the present embodiment along the axial direction.
Fig. 14 is an exploded perspective view showing the cartridge according to the present embodiment.
Fig. 15 is a perspective view showing the tank according to the present embodiment viewed from the opening portion side.
Fig. 16 is a perspective view showing a heater retainer according to the present embodiment viewed from the power unit side.
Fig. 17 is a perspective view showing an atomization container according to the present embodiment viewed from a mesh body side.
Fig. 18 is a front view showing a suction device according to the present embodiment.
Fig. 19 is a cross-sectional view along the axial direction when the mouthpiece is detached from the suction device.
Fig. 20 is a descriptive view showing a state when the cartridge climbs on a vertical engagement convex portion.
Fig. 21 is a descriptive view showing a state of screwing the mouthpiece during the climb-on state of the cartridge.
Fig. 22 is a descriptive view showing a state when the mouthpiece and the cartridge are rotated together.
Fig. 23 is a descriptive view showing a state when the mouthpiece is finally tightened.
Fig. 24 is an enlarged cross-sectional view showing a portion corresponding to the atomization container of the cartridge according to a first modification example of the present embodiment.
Fig. 25 is an enlarged cross-sectional view showing a portion corresponding to the atomization container of the cartridge according to a second modification example of the present embodiment.
Fig. 26 is an enlarged cross-sectional view showing a portion corresponding to the atomization container of the cartridge according to a third modification example of the present embodiment.
Fig. 27 is an enlarged cross-sectional view showing a portion corresponding to the atomization container of the cartridge viewed from a mesh body side according to a fourth modification example of the present embodiment.

### [Description of Embodiments]

Hereinafter, an embodiment of the present invention will be described with reference to figures.

### [Suction device]

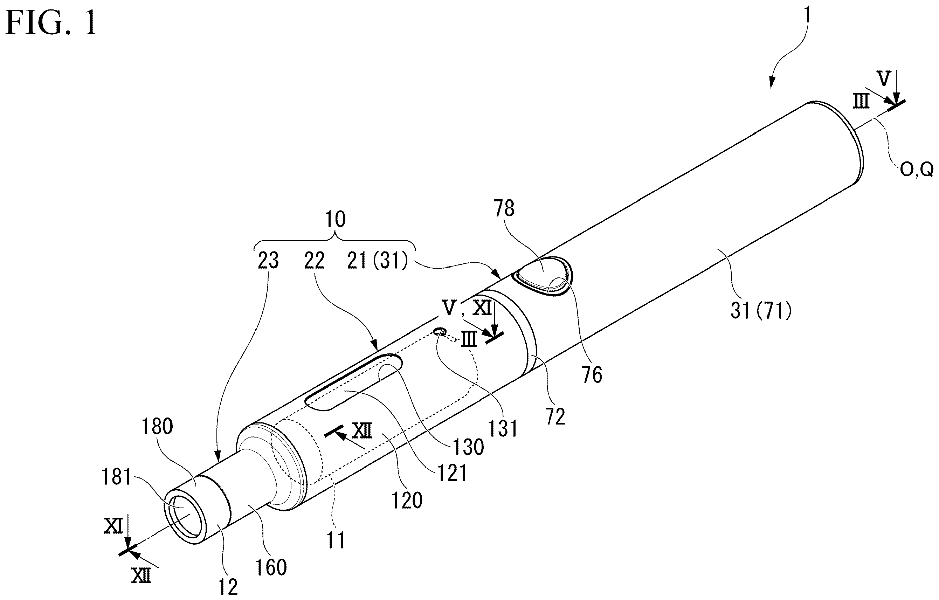

Fig. 1 is a perspective view showing a suction device.

A suction device 1 shown in Fig. 1 is a so-called non-combustion suction device configured for tasting the flavor of tobacco by inhaling aerosol atomized by heating through the tobacco.

The suction device 1 includes a main body unit 10, a tobacco capsule 12, and a cartridge (also referred as an atomization unit) 11 attached to the main body unit 10 configured to be attachable to and detachable from the main body unit 10.

### [Main body unit]

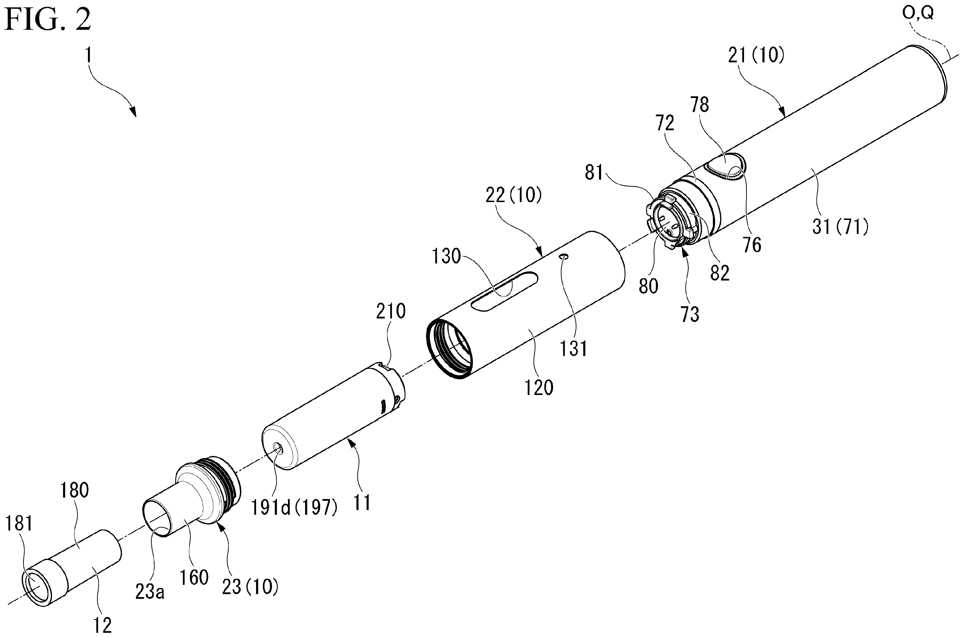

Fig. 2 is an exploded perspective view of the suction device 1.

As shown in Fig. 2, the main body unit 10 includes a power unit 21, a retention unit 22, and a mouthpiece 23. The power unit 21, the retention unit 22, and the mouthpiece 23 are formed in cylindrical shapes with an axis O as a central axis respectively and disposed to be arranged on the axis O. In the following description, the direction along the axis O is described as an axial direction (a normal direction). In this case, in the axial direction, a side from the mouthpiece 23 toward the power unit 21 can be referred to as an opposite-suction-port side or a first end direction side, and a side from the power unit 21 toward the mouthpiece 23 can be referred to as a suction-port side or a second end direction side. A direction intersecting with the axis O in a plan view seen from the axial direction may be referred to as a radial direction, and a direction around the axis O may be referred to as a circumferential direction. In this specification, the recitation "direction" means two directions, and in a case of indicating one of the "directions", the recitation "side" is disclosed.

### [Power unit]

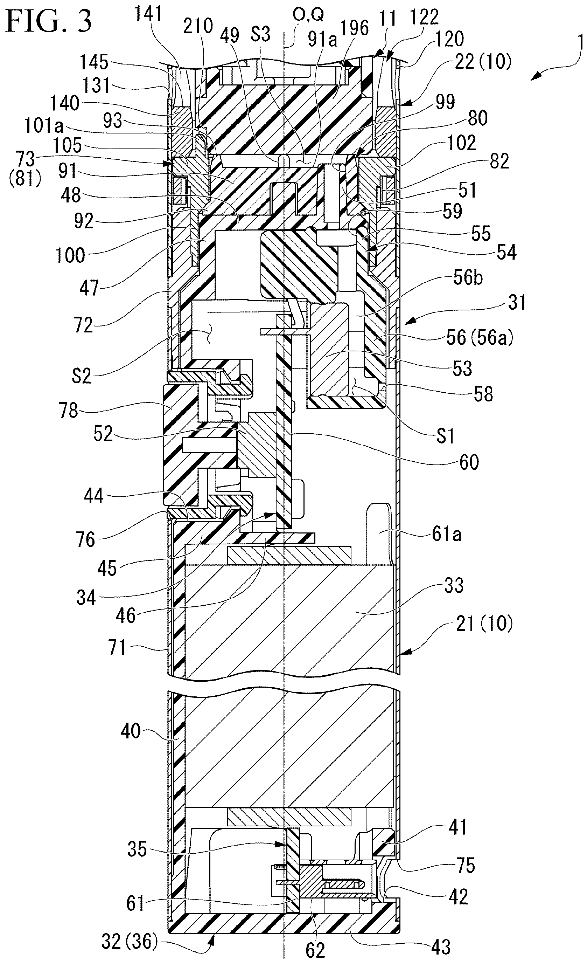

Fig. 3 is a cross-sectional view corresponding to line III-III in Fig. 1.

As shown in Fig. 3, the power unit 21 includes a housing 31 and a holder assembly 32 accommodated in the housing 31.

### [Holder assembly]

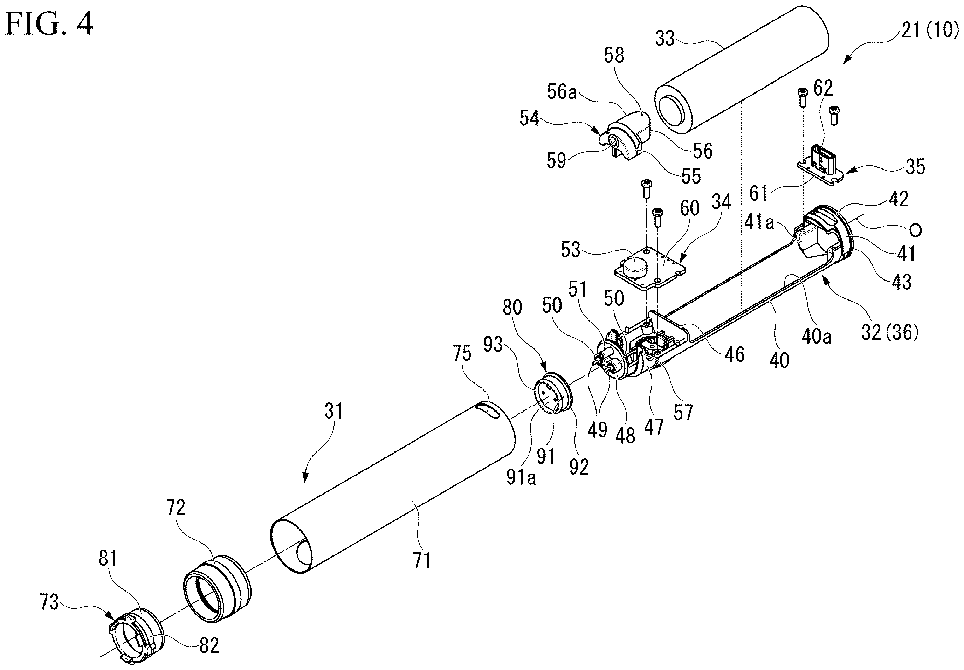

Fig. 4 is an exploded perspective view showing the power unit 21.

As shown in Fig. 3 and Fig. 4, a holder assembly 32 is configured by mounting a storage battery 33, a substrate module (first substrate module 34 and second substrate module 35) and the like on a storage-battery holder 36.

For example, the storage-battery holder 36 is integrally formed from a resin material. The storage-battery holder 36 has a base portion 40. The base portion 40 is formed in a semi-cylindrical shape with the axis O as a central axis. In the base portion 40, if an assembly opening 40a (see Fig. 4) for receiving the storage battery 33 and the like opens outward in the radial direction, the base portion 40 may be formed in a shape besides the semi-cylindrical shape.

In the base portion 40, a press-fit cylindrical portion 41 extends to an end portion at an opposite side with respect to the retention unit 22 in the axial direction. The press-fit cylindrical portion 41 is formed in a cylindrical shape with the axis O as a central axis. In the press-fit cylindrical portion 41, a connector-passage hole 42 penetrating the press-fit cylindrical portion 41 in the radial direction is formed in part of the press-fit cylindrical portion 41 in the circumferential direction. In the press-fit cylindrical portion 41, an opening portion positioned at an opposite side with respect to the retention unit 22 in the axial direction is blocked by a blocking portion 43. The blocking portion 43 is formed in a circular shape having a larger diameter than that of the press-fit cylindrical portion 41.

A button opening 44 (see Fig. 3) is formed in a part of the base portion 40 positioned at the retention unit 22 side in the axial direction. The button opening 44 penetrates part of the base portion 40 in the circumferential direction of the base portion 40 in the radial direction. For example, the above-described connector-passage hole 42 and the button opening 44 are arranged in different positions at 180 degrees in the circumferential direction. According to the present embodiment, the radial direction through each center of the connector-passage hole 42 and the button opening 44 arranged in the circumferential direction is referred to as a front-rear direction. In this case, the connector-passage hole 42 side with respect to the axis O is referred to as a rear side, and the button opening 44 side with respect to the axis O is referred to as a front side. The positions of the connector-passage hole 42 and the button opening 44 may be suitable changed.

In the base portion 40, a button-guide tube 45 extending to the rear side is formed in an opening edge of the button opening 44. The button-guide tube 45 surrounds the circumference of the button opening 44.

In the base portion 40, a partition wall 46 configured to partition the base portion 40 in the axial direction is formed in a portion positioned at the opposite side of the retention unit 22 more than the button opening 44 in the axial direction.

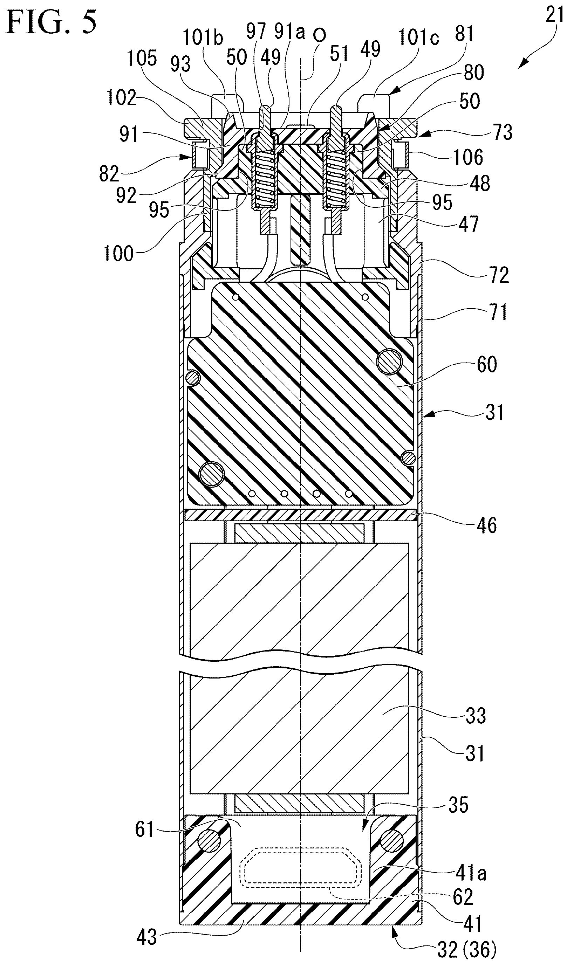

Fig. 5 is a cross-sectional view corresponding to line V-V in Fig. 1.

As shown in Fig. 3 to Fig. 5, a step portion 47 communicates with an end portion positioned at the retention unit 22 side of the base portion 40 in the axial direction. The step portion 47 is formed in a semi-cylindrical shape to be coaxial with the base portion 40, and a distance from the axis O in the radial direction gradually decreases as approaching the retention unit 22 in the axial direction. A connection pedestal 48 communicates with an end edge positioned at the retention unit 22 side in the axial direction in the step portion 47. The connection pedestal 48 is formed in a circular shape with the axis O as a central axis. A pair of electrode retainers 50 and a communication port 51 are formed in the connection pedestal 48.

As shown in Fig. 4 and Fig. 5, the pair of electrode retainers 50 are formed in tubular shapes protruding toward the retention unit 22 in the axial direction. The pair of the electrode retainers 50 are positioned at two sides of the axis O in the radial direction. According to the present embodiment, the pair of the electrode retainers 50 are arranged in a direction (hereinafter, may be referred to as a left-right direction) orthogonal to the above-described front-rear direction among the radial directions. Each electrode retainer 50 extends in the axial direction and is communicated with each other in the radial direction.

As shown in Fig. 3 and Fig. 4, the communication port 51 protrudes from a portion that is positioned at the rear side in the radial direction with respect to the axis O in the connection pedestal 48 toward the retention unit 22 side in the axial direction.

As shown in Fig. 5, a pin electrode 49 is individually held by each electrode retainer 50. The pin electrode 49 is configured from a pin-shaped electrode main body that is elastically supported in a tubular case. The pin electrode 49 is configured that the electrode main body penetrates the electrode retainer 50 in the axial direction in a state in which the tubular case is fitted into the electrode retainer 50. In two end portions of the pin electrode 49 (electrode main body) in the axial direction, the end portion positioned at the opposite side of the retention unit 22 in the axial direction is connected to a first substrate 60 via electrode wirings in the storage-battery holder 36.

The storage battery 33 is formed in a cylindrical shape with the axis O as the axial direction. The storage battery 33 is accommodated in a portion in the base portion 40 that is positioned at the opposite side of the retention unit 22 in the axial direction with respect to the partition wall 46. A power source included in the suction device 1 as a rechargeable and dischargeable power source is not be limited to a secondary battery such as the storage battery 33 and the like and may be a supercapacitor and the like. The power source may be a primary battery.

As shown in Fig. 3 and Fig. 4, the first substrate module 34 is disposed in a part of the base portion 40 positioned at the retention unit 22 side in the axial direction with respect to the partition wall 46. More specifically, the first substrate module 34 has a first substrate 60, a switching element 52 (see Fig. 3), and a pressure sensor 53.

The first substrate 60 is configured to have the front-rear direction as a thickness direction. More specifically, the first substrate 60 is fixed to the base portion 40 by screws and the like in a state of being placed on an opening end surface of the assembly opening 40a. The first substrate 60 is connected to the storage battery 33 via a first connection wiring (not shown). In the example shown in Fig. 3, the first substrate 60 is positioned on the axis O.

The switching element 52 is disposed at a position overlapping the button opening 44 on a front surface (first principal plane) of the first substrate 60 when viewed from the front-rear direction. According to the present embodiment, the switching element 52 is surface mounted on the first substrate 60. However, the switching element 52 may be mounted on the first substrate 60 in a state in which a connection terminal extending from the switching element 52 is inserted through the penetration hole of the first substrate 60.

The pressure sensor 53 is disposed at the retention unit 22 side with respect to the switching element 52 in the axial direction on a rear surface (second principal plane) of the first substrate 60. In other words, the pressure sensor 53 is disposed at a position that does not overlap the switching element 52 in a planar view in the front-rear direction. According to the present embodiment, the pressure sensor 53 is disposed at the position shifting to the retention unit 22 side in the axial direction with respect to the switching element 52; however, the configuration is not limited thereto. In other words, if the switching element 52 and the pressure sensor 53 are disposed at misaligned positions in the in-plane direction of the first substrate 60, the switching element 52 and the pressure sensor 53 may be disposed at misaligned positions at the opposite side of the retention unit 22 in the axial direction and may be disposed at misaligned positions in the left-right direction among the radial directions.

The pressure sensor 53 may be configured by adopting an electrostatic capacitance type sensor for example. In other words, the pressure sensor 53 is configured to detect behavior of diaphragm deforming in response to pressure change as the change of electrostatic capacitance. The pressure sensor 53 according to the present embodiment is mounted on the first substrate 60 in the state in which a connection terminal extending from the pressure sensor 53 is inserted through the penetration hole of the first substrate 60. However, the pressure sensor 53 may be surface mounted on the first substrate 60.

A sensor holder 54 is attached to the pressure sensor 53. The sensor holder 54 is formed from a resin material such as a silicone resin and the like that is softer than the storage-battery holder 36 and has elasticity. The sensor holder 54 has an attachment portion 55 being attached to the storage-battery holder 36 and a cover 56 for covering the pressure sensor 53.

The attachment portion 55 is formed in a semicircular shape. The attachment portion 55 is assembled to the storage-battery holder 36 in a state of being abut by the above-described connection pedestal 48 from the opposite side of the retention unit 22 in the axial direction. A clipping piece 57 (see Fig. 4) is formed in the step portion 47 and configured to retain the attachment portion 55 in the space between the connection pedestal 48 and the step portion 47 in the axial direction. The clipping piece 57 protrudes from two end surfaces of a circular arc in the circumferential direction, wherein the circular arc is positioned at an external side in the radial direction (left-right direction) of the step portion 47.

The cover 56 communicates with the attachment portion 55 at the opposite side of the retention unit 22 in the axial direction. The cover 56 is formed in a cap shape that opens at the front side. A spacer 56b swelling toward the front side is formed in a bottom wall portion 56a of the cover 56. The pressure sensor 53 is fitted into the cover 56 in a state of being abut by the spacer 56a. Accordingly, a gap in the radial direction is formed between the internal surface of the bottom wall portion 56a and the pressure sensor 53. An air replacement hole 58 penetrating the bottom wall portion 56a in the radial direction is formed in the bottom wall portion 56a.

A communication passage 59 communicating the inside of the communication port 51 and the inside of the cover 56 is formed in the above-described attachment portion 55. The communication passage 59 extends along the axial direction in the attachment portion 55. An end portion of the communication passage 59 at the opposite side of the retention unit 22 in the axial direction opens on the internal circumferential surface of the cover 56. On the other hand, an end portion of the communication passage 59 at the retention unit 22 side in the axial direction opens on a surface facing the retention unit 22 side in the axial direction in the attachment portion 55. According to the present embodiment, a minimum inner diameter of the communication passage 59 is larger than a maximum inner diameter of the air replacement hole 58. In the communication passage 59, at least the inner diameter of the end portion at the retention unit 22 side in the axial direction is larger than the inner diameter of the communication port 51.

According to the present embodiment, the communication port 51 and the communication passage 59 are disposed at a position where at least part of the communication port 51 and the communication passage 59 overlaps the pressure sensor 53 when viewed in the axial direction. However, the communication port 51 and the communication passage 59 may be disposed at a position shifting from the pressure sensor 53 when viewed in the axial direction.

As shown in Figs. 3-5, the second substrate module 35 is disposed at the opposite side of the first substrate module 34 in the axial direction to sandwich the storage battery 33 therebetween. In other words, the substrate modules 34, 35 according to the present embodiment are disposed at two sides in the axial direction to sandwich the storage battery 33 therebetween. The second substrate module 35 has a second substrate 61 and a female connector 62.

The second substrate 61 is accommodated in the press-fit cylindrical portion 41 having the radial direction (front-rear direction) as the thickness direction. As shown in Fig. 5, the second substrate 61 is fixed to a boss portion 41a by screws in a state of being placed on the boss portion 41a, wherein the boss portion 41a protrudes inwardly from the press-fit cylindrical portion in the radial direction. The second substrate 61 is connected to the first substrate 60 via a second wiring 61a. In other words, the second wiring 61a is drawn to pass through the circumference of the storage battery 33 in the axial direction at the external side of the storage-battery holder 36.

As shown in Figs. 3-4, the female connector 62 is used as a configuration for the power charge of the storage battery 33, and a male connector (not shown) drawn from an external power source is inserted into and pulled from the female connector 62. According to the present embodiment, for example, a USB (Universal Serial Bus) connector is adopted as the female connector 62. However, the female connector 62 is not limited to the USB connector. The female connector 62 is not necessary to be used for the power charge and may be used as a configuration for communication, for example.

The female connector 62 is implemented on the second substrate 61 in a state in which the opening portion faces the rear side. A tip end portion (an end portion close to the opening portion) of the female connector 62 is inserted into the connector-passage hole 42. However, the female connector 62 may be retracted to the internal side from the connector-passage hole 42 in the radial direction.

### (Housing)

As shown in Figs. 3-4, the housing 31 has an exterior cylindrical portion 71, an intervenient member 72, and a connection mechanism 73.

The exterior cylindrical portion 71 is formed in a cylindrical shape having the axis O as a central axis. The holder assembly 32 is inserted into the exterior cylindrical portion 71 through an opening portion positioned at the opposite side of the retention unit 22 in the axial direction. More specifically, the holder assembly 32 is assembled to the exterior cylindrical portion 71 in a state in which the press-fit cylindrical portion 41 of the storage battery 36 is pressed to fit into an end portion of the exterior cylindrical portion 71 positioned at the opposite side of the retention unit 22. Accordingly, the holder assembly 32 is accommodated into the exterior cylindrical portion 71 in a state in which an end portion positioned at the retention unit 22 side protrudes from the exterior cylindrical portion 71. An opening portion of the exterior cylindrical portion 71 positioned at the opposite side of the retention unit 22 in the axial direction is blocked by the blocking portion 43 of the storage-battery holder 36.

A connector exposure hole 75 is formed in a portion overlapping the connector-passage hole 42 and the female connector 62 viewed in the radial direction in the end portion of the exterior cylindrical portion 71 positioned at the opposite side of the retention unit 22 in the axial direction. The connector exposure hole 75 penetrates the exterior cylindrical portion 71 in the radial direction. According to the present embodiment, a configuration that the female connector 62 opens in the radial direction is described, the female connector 62 may open in the axial direction.

A button exposure hole 76 is formed in a portion overlapping the button opening 44 viewed in the radial direction in the end portion of the exterior cylindrical portion 71 at the retention unit 22 side. The button exposure hole 76 penetrates the exterior cylindrical portion 71 in the radial direction.

The button 78 is accommodated in the button exposure hole 76 and the button opening 44. The button 78 is configured to be movable in the radial direction in a state of being supported by the button-guide tube 45. The button 78 operates to press the switch element 52 while moving inward in the radial direction. A surface of the button 78 is exposed to an external circumferential surface of the exterior cylindrical portion 71 through the button exposure hole 76. The button 78 is not limited to move in the radial direction, for example, the button 78 may be configured to slide in the axial direction. A configuration operating the suction device 1 by a touch sensor or the like instead of the button 78 may be configured.

The intervenient member 72 is formed in a cylindrical shape with the axis O as a central axis. The intervenient member 72 is fitted into an interval between the holder assembly 32 and the exterior cylindrical portion 71 from the retention unit 22 side in the axial direction. Accordingly, a portion between the holder assembly 32 and the exterior cylindrical portion 71 is sealed in the opening portion of the exterior cylindrical portion 71 positioned at the retention unit 22 side in the axial direction.

As shown in Fig. 3, a space surrounded by the sensor holder 54 in the housing 31 configures a pressure change room S1 in which a pressure changes through the communication port 51 in response to the usage (suction) of the suction device 1. On the other hand, in the housing 31, space other than the pressure change room S1 configures a constant pressure room S2 in which the atmospheric pressure applies. According to the present embodiment, among the storage battery 33 and the substrate modules 34, 35, the configurations other than the pressure sensor 53 are accommodated in the constant pressure room S2. However, if at least the pressure sensor 53 is accommodated in the pressure change room S 1, components other than the pressure sensor 53 may be accommodated in the pressure change room S1. In the housing 31, a liquid detection seal and the like may be provided so as to understand infiltration of the liquid.

### (Connection mechanism)

As shown in Fig. 4 and Fig. 5, the connection mechanism 73 has a connection cap 80, a first connection member 81, and an annular piece 82.

The connection cap 80 is formed from a resin material being softer than the storage-battery holder 36 and having elasticity such as the silicone resin and the like. The connection cap 80 is attached to the connection pedestal 48 from the retention unit 22 side in the axial direction. The connection cap 80 has a base portion 91, a flange portion 92, and a surrounding convex portion 93.

As shown in Fig. 5, the base portion 91 is formed in a cylindrical shape having the axis O as a central axis. In the base portion 91, accommodation concave portions 95 recessed toward the retention unit 22 side in the axial direction are formed in positions overlapping each electrode retainer 50 in the planar view respectively. Each accommodation concave portion 95 extends in the axial direction and the accommodation concave portions 95 are communicated in the radial direction. In the base portion 91, an electrode insertion hole 97 is formed at the position overlapping each accommodation concave portion 95 in the planar view. The electrode insertion hole 97 penetrates the base portion 91 in the axial direction and communicates with the inside of the accommodation concave portion 95.

As shown in Fig. 3, in the base portion 91, a port insertion hole 99 is formed at the position overlapping the communication port 51 in the planar view. The port insertion hole 99 penetrates the base portion 91 in the axial direction.

As shown in Fig. 3 and Fig. 5, in the connection cap 80, the electrode retainer 50 is accommodated in each accommodation concave portion 95, and the communication port 51 is inserted into the port insertion hole 99. Accordingly, the connection cap 80 is assembled with the storage-battery holder 36 in a state of abutting with an end surface of the connection pedestal 48 facing the retention unit 22 side in the axial direction. In such state, the pin electrode 49 protrudes toward the retention unit 22 side in the axial direction from the base portion 91 and through the electrode insertion hole 97. The communication port 51 protrudes toward the retention unit 22 side in the axial direction from the base portion 91 and through the port insertion hole 99. In other words, the surface facing the retention unit 22 side in the connection cap 80 (base portion 91) forms a base surface 91a from which the pin electrode 49 protrudes and where the communication port 51 opens.

The flange portion 92 expands outwardly in the radial direction in the end portion of the base portion 91 at the opposite side of the retention unit 22 in the axial direction.

The surrounding convex portion 93 protrudes in the axial direction from the end surface of the base portion 91 facing the retention unit 22 side in the axial direction. More specifically, the surrounding convex portion 93 is formed in an annular shape extending along an external circumferential edge of the base portion 91. In other words, the surrounding convex portion 93 is configured to surround the pin electrode 49 and the communication port 51 together at a separated position at the external side in the radial direction with respect to the pin electrode 49 and the communication port 51. If the surrounding convex portion 93 is the configuration to surround the circumference of the pin electrode 49 and the communication port 51 together, the surrounding convex portion 93 may be positioned at an internal side in the radial direction with respect to the external circumferential edge of the base portion 91. The surrounding convex portion 93 is not limited to the annular shape and may be formed in a polygonal shape or the like. According to the present embodiment, the phrase "surrounding" is not limited to a configuration extending continuously and also includes the configuration extending intermittently. In other words, the surrounding convex portion 93 according to the present embodiment may be suitably changed if the surrounding convex portion 93 is the configuration surrounding the circumference of the pin electrode 49 and the communication port 51 together.

The surrounding convex portion 93 is formed in a triangle shape having a sharp tip end toward the retention unit 22 side in the axial direction in a vertical cross-sectional view along the axial direction. A protrusion height of the surrounding convex portion 93 from the base portion 91 is higher than the communication port 51 and lower than the pin electrode 49. However, the protrusion height of the surrounding convex portion 93 may be higher than the pin electrode 49. The shape of the surrounding convex portion 93 in the vertical cross-sectional view is not limited to the triangle shape.

The first connection member 81 has a base cylindrical portion 100, a vertical engagement convex portion (from first vertical engagement convex portion 101a to third vertical engagement convex portion 101c), and a horizontal engagement convex portion 102.

The base cylindrical portion 100 is formed in a multi-stage cylindrical shape having the axis O as a central axis, and a dimeter decreases by steps toward the retention unit 22 side in the axial direction. An end portion in the base cylindrical portion 100 positioned at the opposite side of the retention unit 22 in the axial direction is fitted into the internal side of the intervenient member 72. In this state, an end portion in the base cylindrical portion 100 at the retention unit 22 side in the axial direction surrounds the circumference of the connection cap 80 in a state of sandwiching the flange portion 92 in an interval with the connection pedestal 48 in the axial direction. An external flange portion 105 expanding outwardly in the radial direction is formed in the end portion in the base cylindrical portion 100 at the retention unit 22 side in the axial direction.

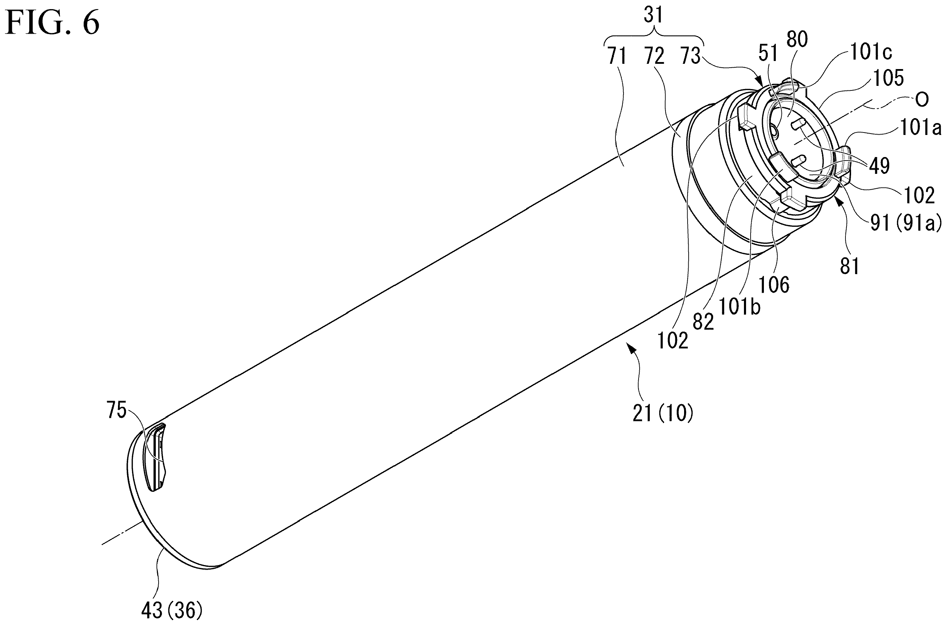

Fig. 6 is a perspective view of the power unit 21.

As shown in Fig. 5 and Fig. 6, the vertical engagement convex portions 101a-101c protrude toward the retention unit 22 side in the axial direction from the base cylindrical portion 100. A plurality of the vertical engagement convex portions 101a-101c are formed to be separated at intervals in the circumferential direction. According to the present embodiment, each of the vertical engagement convex portions 101a-101c are evenly disposed in the circumferential direction by a 120-degree interval. The vertical engagement convex portions 101a-101c may be single or multiple. A pitch of the vertical engagement convex portions 101a-101c may be suitably changed. In this case, the multiple vertical engagement convex portions 101a-101c may be unevenly disposed.

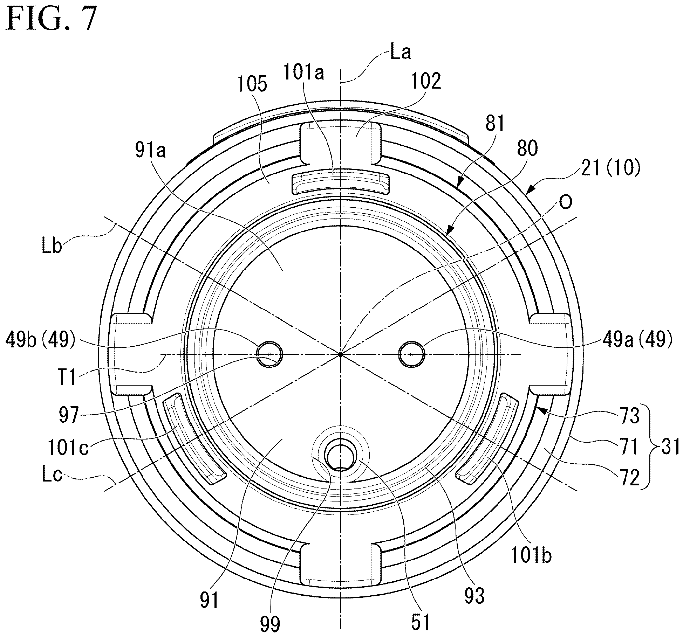

Fig. 7 is a planar view showing the power unit 21 viewed from the retention unit 22 side.

As shown in Fig. 7, each of the vertical engagement convex portions 101a-101c is disposed so as to cause the pin electrode 49 not to be disposed on virtual straight lines La-Lc connecting the center in the circumferential direction of each vertical engagement convex portion 101a-101c and the axis O. More specifically, the pin electrodes 49 are disposed at positions being line symmetry with respect to the virtual straight line La connecting the first vertical engagement convex portion 101a and the axis O. In other words, a virtual straight line T1 connecting each pin electrode 49 is orthogonal to the virtual straight line La and distances from the virtual straight line La to each pin electrode 49 are the same as each other.

As shown in Fig. 5 and Fig. 6, an end edge in each vertical engagement convex portion 101a-101c positioned at the retention unit 22 side in the axial direction is positioned at the retention unit 22 side in the axial direction more than the pin electrode 49. Each vertical engagement convex portion 101a-101c is formed in a rectangle shape in a side view from the radial direction respectively. In an end portion at the retention unit 22 side in the axial direction in each vertical engagement convex portion 101a-101c, a surface facing the internal side in the radial direction is formed as an inclined surface whose thickness in the radial direction gradually becomes thinner toward the retention unit 22 side in the axial direction. The inclined surface functions as a guide for smoothly guiding each vertical engagement convex portion 101a-101c to an engagement concave portion 210 of the cartridge 11 described below.

The horizontal engagement convex portion 102 protrudes outwardly in the radial direction from the external flange portion 105. The horizontal engagement convex portion 102 is formed in a rectangle shape in the planar view. A plurality of the horizontal engagement convex portions 102 are formed to be separated by intervals in the circumferential direction. According to the present embodiment, each of the horizontal engagement convex portions 102 is evenly disposed in the circumferential direction by a 90-degree interval. According to the present embodiment, a single horizontal engagement convex portion 102 is disposed at the same position with the first vertical engagement convex portion 101a in the circumferential direction. The horizontal engagement convex portion 102 may be single or multiple. A pitch of the horizontal engagement convex portions 102 may be suitably changed. In this case, multiple horizontal engagement convex portions 102 may be unevenly disposed.

The annular piece 82 is formed in a thin annular shape. The base cylindrical portion 100 is inserted into the annular piece 82 from the retention unit 22 side in the axial direction such that the annular piece 82 is clipped between the intervenient member 72 and the external flange portion 105 in the axial direction. As shown in Fig. 5, a bending portion 106 is formed in a portion of the annular piece 82 in the circumferential direction. The bending portion 106 is formed in an arch shape expanding outwardly in the radial direction. The bending portion 106 is configured to be elastically deformable in the radial direction. The bending portion 106 is positioned at the internal side in the radial direction more than an external end surface of the horizontal engagement convex portion 102.

A plurality of the bending portions 106 are formed to be separated by intervals in the circumferential direction. For example, the bending portions 106 are disposed at the same positions in the circumferential direction of a pair of horizontal engagement convex portions 102 that are opposed with each other in the radial direction (left-right direction) among the horizontal engagement convex portions 102. However, a number of the bending portions 106 may be suitably changed. For example, the bending portion 106 may be formed corresponding to each horizontal engagement convex portion 102, or the bending portion 106 may be formed corresponding to only one horizontal engagement convex portion 102.

### (Retaining unit)

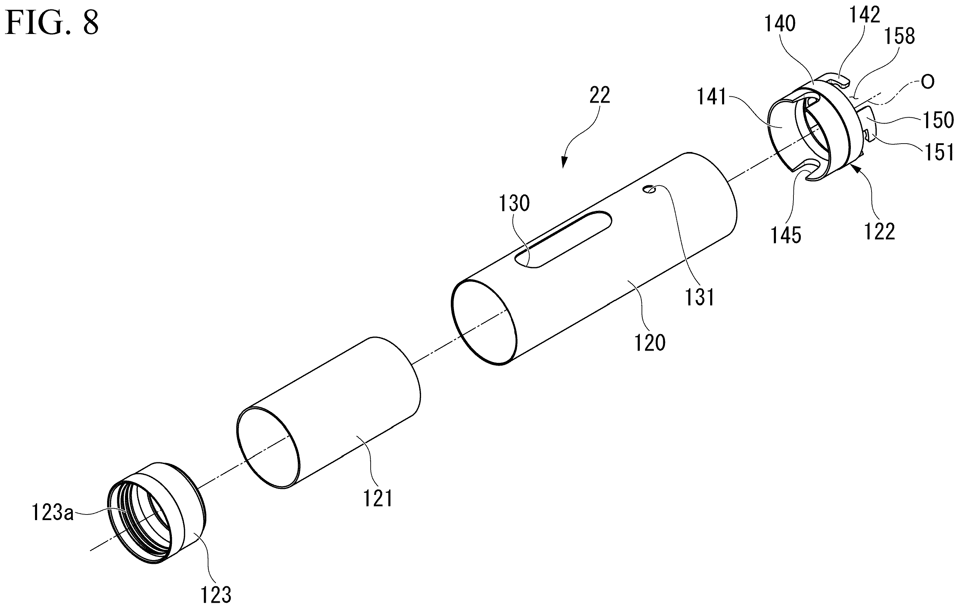

Fig. 8 is an exploded perspective view of the retention unit 22.

As shown in Fig. 8, the retention unit 22 is attached to the main body unit 10 so as to be attachable to and detachable from the main body unit 10. More specifically, the retention unit 22 has a container-retaining cylinder 120, a transmission cylinder 121, a second connection member 122, and a sleeve 123.

The container-retaining cylinder 120 is formed in a cylindrical shape with the axis O as a central axis. An observation hole 130 is formed in a central portion of the container-retaining cylinder 120 in the axial direction. The observation hole 130 penetrates the container-retaining cylinder 120 in the radial direction. The observation hole 130 is formed in an oval shape with the axial direction as a longitudinal direction. The observation hole 130 is formed in a portion of the container-retaining cylinder 120 being opposed with each other in the radial direction. A number, a position, a shape and the like of the observation hole 130 may be suitably changed.

A ventilation hole 131 is formed in a portion of the container-retaining cylinder 120 positioned at the power unit 21 side in the axial direction more than the observation hole 130. The ventilation hole 131 penetrates the container-retaining cylinder 120 in the radial direction. The ventilation hole 131 causes the inside and outside of the retention unit 22 to be communicated with each other. The ventilation hole 131 is formed in a portion of the container-retaining cylinder 120 being opposed with each other in the radial direction (front-rear direction). A number, a position, a shape and the like of the ventilation hole 131 may be suitably changed.

The transmission cylinder 121 is formed from a material having optical transparency. The transmission cylinder 121 is inserted into the container-retaining cylinder 120. More specifically, the transmission cylinder 121 is positioned at the mouthpiece 23 side in the axial direction more than the ventilation hole 131 in the container-retaining cylinder 120 to cover the observation hole 130 from the internal side in the radial direction. In other words, the user can visually recognize the inside of the retention unit 22 through the observation hole 130 and the transmission cylinder 121. The retention unit 22 may be configured without the observation hole 130 and the transmission cylinder 121.

The second connection member 122 is locked by the first connection member 81 at the time of attaching the retention unit 22 to the main body unit 10. More specifically, the second connection member 122 has a fitting cylinder 140, a guide cylinder 141, and a locking piece 142.

The fitting cylinder 140 is formed in a cylindrical shape with the axis O as a central axis. The fitting cylinder 140 is fitted into a portion of the container-retaining cylinder 120 positioned at the power unit 21 side in the axial direction more than the transmission cylinder 121 by press fitting or the like.

The guide cylinder 141 is disposed to be coaxial with the fitting cylinder 140. The guide cylinder 141 extends to the mouthpiece 23 side in the axial direction from the fitting cylinder 140. The guide cylinder 141 is formed in a tapered cylindrical shape whose internal diameter gradually increases toward the mouthpiece 23 side in the axial direction. An external diameter of the guide cylinder 141 is smaller than an external diameter of the fitting cylinder 140. In the guide cylinder 141, a clearance portion 145 is formed at a position overlapping the ventilation hole 131 in a side view viewed from the radial direction. For example, the clearance portion 145 is formed in a U shape having an opening at the mouthpiece 23 side in the axial direction. The ventilation hole 131 opens to the inside of the retention unit 22 through the clearance portion 145. The shape of the clearance portion 145 only has to be configured to cause at least part of the ventilation hole 131 to be exposed in the retention unit 22. In a case in which the guide cylinder 141 and the ventilation hole 131 are disposed in different positions in the axial direction, the guide cylinder 141 may be configured without the clearance portion 145.

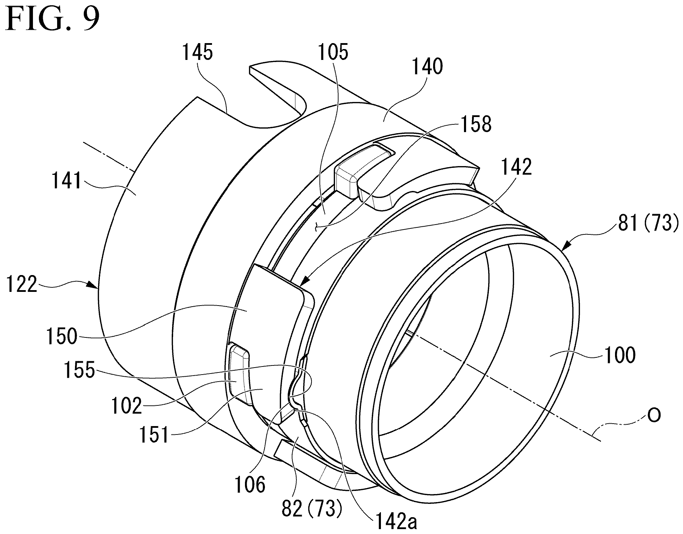

Fig. 9 is a perspective view showing a connection structure of the first connection member 81 and the second connection member 122.

As shown in Fig. 8 and Fig. 9, the locking piece 142 protrudes toward the power unit 21 side in the axial direction from the fitting cylinder 140. The locking piece 142 is formed in a L shape in a side view viewed from the radial direction. More specifically, the locking piece 142 has a vertical extending portion 150 and a horizontal extending portion 151.

The vertical extending portion 150 protrudes toward the power unit 21 side in the axial direction from the fitting cylinder 140.

As shown in Fig. 9, the horizontal extending portion 151 extends from an end portion of the vertical extending portion 150 at the power unit 21 side toward one side only in the circumferential direction.

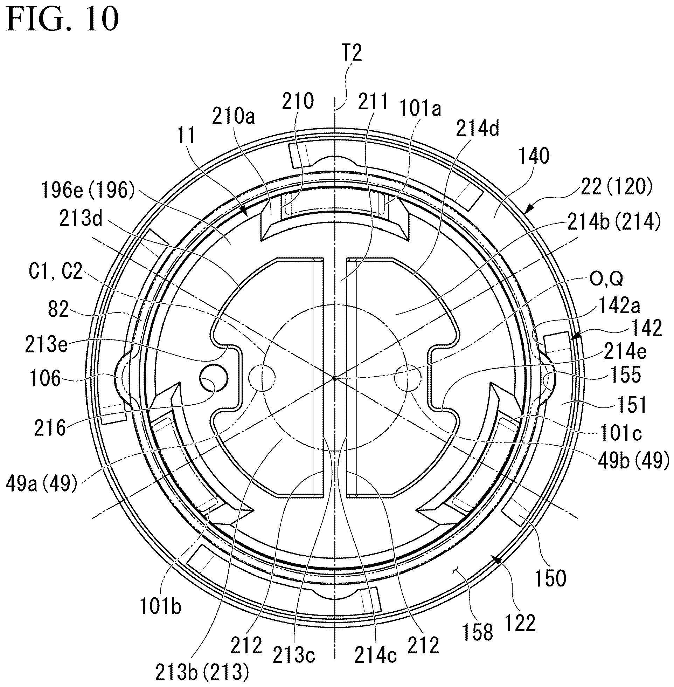

Fig. 10 is a planar view of the retention unit 22 and the cartridge 11 viewed from the power unit 21 side in the axial direction.

As shown in Fig. 9 and Fig. 10, in the horizontal extending portion 151, an engagement concave portion 155 recessed toward the external side in the radial direction is formed in the end portion at the one side of the circumferential direction. The engagement concave portion 155 is formed in a semicircular shape toward the external side in the radial direction.

A plurality of the locking pieces 142 are formed to be separated by intervals in the circumferential direction. According to the present embodiment, each of the locking pieces 142 is evenly disposed in the circumferential direction by a 90-degree interval. Between two adjacent locking pieces 142 in the circumferential direction, an engagement groove 158 is formed for the horizontal engagement convex portion 102 to be inserted. The engagement groove 158 is formed in an L shape in the side view.

As shown in Fig. 2 and Fig. 9, the power unit 21 and the retention unit 22 are configured to be attachable and detachable by connecting the locking piece 142 and the horizontal engagement convex portion 102. In other words, in order to connect the power unit 21 and the retention unit 22, the horizontal engagement concave portion 102 is inserted into the engagement groove 158 in the axial direction, and then the power unit 21 and the retention unit 22 are relatively rotated around the axis O. Accordingly, the horizontal engagement concave portion 102 is engaged between the horizontal extending portion 151 and the fitting cylinder 140 in the axial direction. During the procedure when the power unit 21 and the retention unit 22 are relatively rotated around the axis O, the bending portion 106 of the annular piece 82 are fitted into the engagement concave portion 155. Accordingly, the bending portion 106 is engaged with the engagement concave portion 155 in the circumferential direction. As a result, the power unit 21 and the retention unit 22 are assembled with each other in a state in which position alignment in the axial direction and the circumferential direction is finished.

As shown in Fig. 9, in the engagement groove 158 according to the present embodiment, a portion between the fitting cylinder 140 and the horizontal extending portion 151 is formed in a tapered shape with a width in the axial direction that gradually becomes narrower from the other side toward the one side in the circumferential direction. More specifically, an end surface of the horizontal extending portion 151 facing the mouthpiece 23 side in the axial direction is formed in an inclined surface extending toward the power unit 21 side in the axial direction from the other side toward the one side in the circumferential direction.

The horizontal engagement convex portion 102 is formed in a tapered shape with a width in the axial direction that gradually becomes narrower from the other side toward the one side in the circumferential direction. More specifically, an end surface of the horizontal engagement convex portion 102 facing the opposite side of the retention unit 22 in the axial direction is formed in an inclined surface extending to the mouthpiece 23 side in the axial direction from the one side toward the other side in the circumferential direction. Accordingly, it is possible to prevent interference of the horizontal extending portion 151 and the horizontal engagement convex portion 102 and improve the assembling workability at the time of connecting the power unit 21 with the retention unit 22.

As shown in Fig. 8, the sleeve 123 is fitted into part of the container-retaining cylinder 120 that is positioned at the mouthpiece 23 side more than the transmission cylinder 121 in the axial direction by being pressed or the like. The transmission cylinder 121 is held in the axial direction between the second connection member 122 and the sleeve 123. A female screw portion 123a is formed on an internal circumferential surface of the sleeve 123.

### (Mouthpiece)

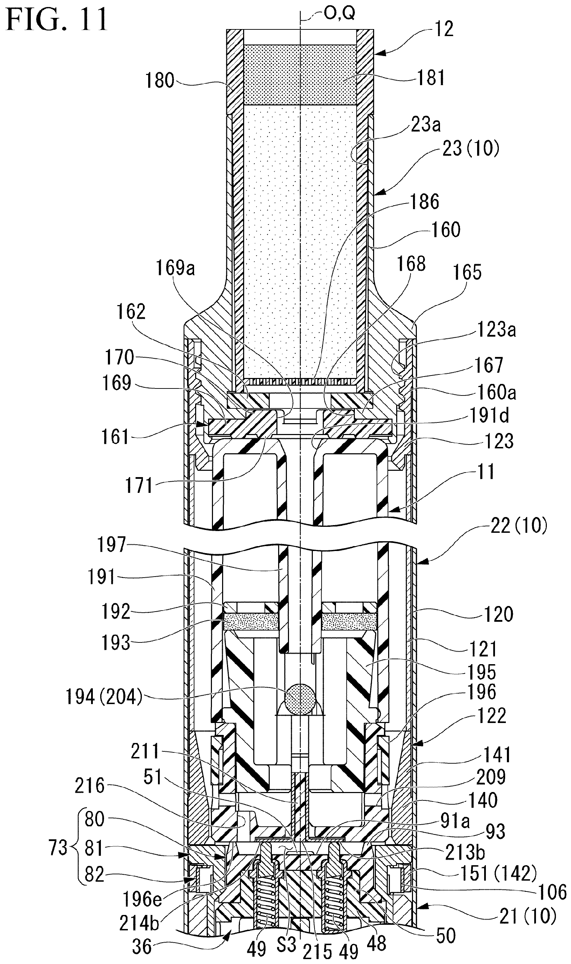

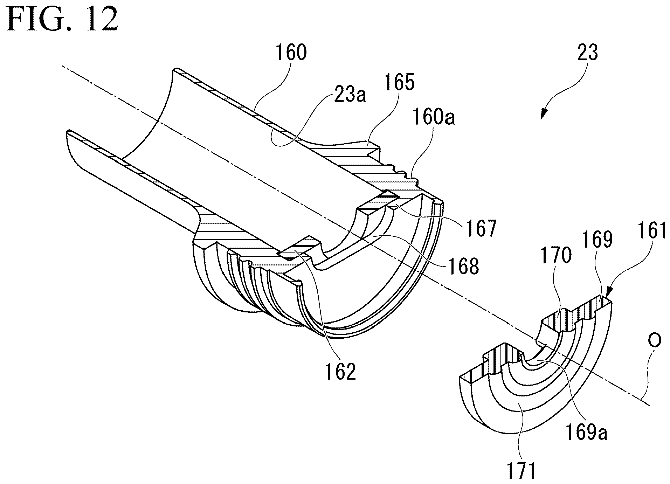

Fig. 11 is a cross-sectional view along line XI-XI in Fig. 1. Fig. 12 is an exploded perspective view of the mouthpiece 23 corresponding to line XII-XII in Fig. 1.

As shown in Fig. 11 and Fig. 12, the mouthpiece 23 has a mouthpiece main body 160 and a slip prevention member (first slip prevention member 161 and second slip prevention member 162).

A suction port 23a being capable of accommodating the tobacco capsule 12 is formed in the mouthpiece 23. The mouthpiece main body 160 is formed in a multi-stage cylindrical shape with the axis O as a central axis. A male screw portion 160a is formed in an end portion of the mouthpiece main body 160 at the retention unit 22 side in the axial direction. The male screw portion 160a of the mouthpiece main body 160 is screwed to the female screw portion 123a of the sleeve 123 to be attachable thereto and detachable therefrom. The mouthpiece main body 160 may be a configuration attaching to or detaching from the sleeve 123 by a method besides the screwing (for example, fitting or the like).

In the mouthpiece main body 160, an abutting flange 165 is formed in a portion positioned at the opposite side of the retention unit 22 in the axial direction with respect to the male screw portion 160a. The abutting flange 165 is formed in an annular shape extending outwardly in the radial direction. The abutting flange abuts on the retention unit 22 in the axial direction in a state in which the mouthpiece 23 is attached to the retention unit 22. The abutting flange 165 is configured such that an external diameter of the abutting flange 165 gradually decreases away from the retention unit 22 in the axial direction.

A partitioning portion 167 configured to partition the inside of the mouthpiece main body 160 in the axial direction is formed in an end portion of the mouthpiece main body 160 at the retention unit 22 side in the axial direction. In the partitioning portion 167, a penetration hole 168 penetrating the partitioning portion 167 is formed at a position overlapping the axis O. For example, the penetration hole 168 is formed in an oval shape having one direction of the radial direction as a longitudinal direction. A shape of the penetration hole 168 in a planar view may be a perfect circle shape, a polygonal shape or the like.

For example, the first slip prevention member 161 is integrally formed from a resin material such as a silicone resin or the like. The first slip prevention member 161 has a ring portion 169, a fitting protrusion 170, and an engagement protrusion 171.

The ring portion 169 is fitted in the mouthpiece main body 160 from the retention unit 22 side in the axial direction. Position alignment of the first slip prevention member 161 in the axial direction with respect to the mouthpiece main body 160 is performed by the ring portion 169 abutting the partitioning portion 167 in the axial direction.

A communication hole 169a is formed in a center of the ring portion 169. The communication hole 169a is formed to cause the inside of the retention unit 22 and the inside of the mouthpiece main body 160 to be communicated via the penetration hole 168.

A pair of the fitting protrusions 170 are formed at positions facing each other in the radial direction and sandwiching the communication hole 169a therebetween in the internal circumferential edge of the ring portion 169. The fitting protrusions 170 protrude toward the opposite side of the retention unit 22 in the axial direction from the ring portion 169. Each of the fitting protrusions 170 is fitted to two end portions of the penetration hole 168 in the radial direction. Accordingly, position alignment of the first slip prevention member 161 with the mouthpiece main body 160 in the circumferential direction is performed. Accordingly to the present embodiment, the configuration that the fitting protrusions 170 are fitted into the penetration hole 168 is described; however, a configuration that the fitting protrusions 170 are fitted into other hole besides the penetration hole 168 may be configured.

The engagement protrusion 171 protrudes toward the retention unit 22 side in the axial direction from the ring portion 169. The engagement protrusion 171 is formed in a circular shape having the axis O as a center. According to the present embodiment, two of the engagement protrusions 171 are formed in a concentric circular shape. The first slip prevention member 161 may be a configuration without the engagement protrusion 171.

For example, the second slip prevention member 162 is integrally formed from the resin material such as the silicone resin or the like. The second slip prevention member 162 is fitted into the mouthpiece main body 160 from the opposite side of the retention unit 22 in the axial direction. The position alignment of the second slip prevention member 162 with respect to the mouthpiece main body 160 in the axial direction is performed by being abutted by the partitioning portion 167 in the axial direction.

### (Tobacco capsule)

As shown in Fig. 2 and Fig. 11, the tobacco capsule 12 is attached into the mouthpiece main body 160 from the opposite side of the retention unit 22 in the axial direction so as to be attachable thereto and detachable therefrom. The tobacco capsule 12 has a capsule portion 180 and a filter portion 181.

As shown in Fig. 11, the capsule portion 180 is formed in a bottomed cylindrical shape having the axis O as a central axis. In the capsule portion 180, in a bottom wall portion 186 for blocking an opening portion at the retention unit 22 side in the axial direction, a mesh opening penetrating the bottom wall portion 186 in the axial direction is formed.

The filter portion 181 is fitted into the capsule portion 180 from the opposite side of the retention unit 22 in the axial direction. Tobacco is sealed in a space formed by the capsule portion 180 and the filter portion 181.

### (Cartri dge)

As shown in Fig. 2, the cartridge 11 is configured to store the liquid aerosol source while atomizing the liquid aerosol source. The cartridge 11 is accommodated in the transmission cylinder 121 of the retention unit 22.

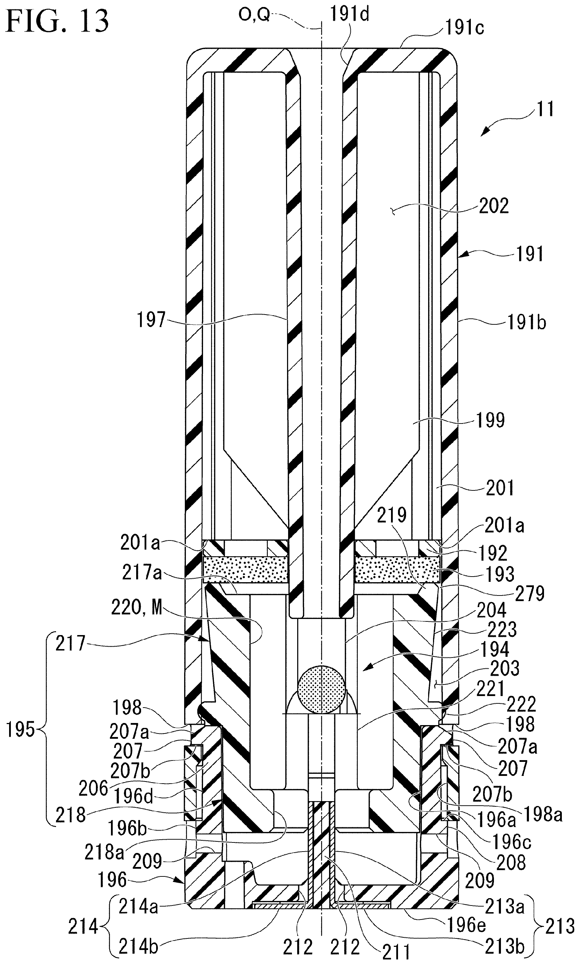

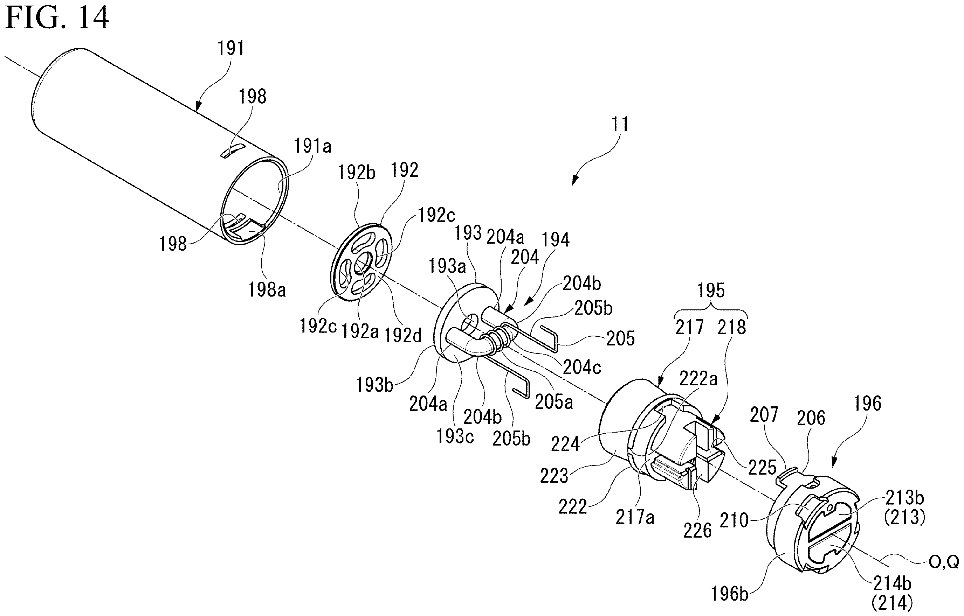

Fig. 13 is a cross-sectional view of the cartridge 11 along the axial direction. Fig. 14 is an exploded perspective view of the cartridge 11.

As shown in Fig. 13 and Fig. 14, the cartridge 11 has a tank 191 formed in a bottomed cylindrical shape, a gasket 192 formed in a substantially disc shape and accommodated in the tank 191, a mesh body (also referred to as a partition plate) 193 formed in a substantially disc shape, a heater 194, an atomization container (also referred to as an elastic applying member) 195, and a heater holder (also referred to as a holder) 196 configured to block an opening portion 191a of the tank 191.

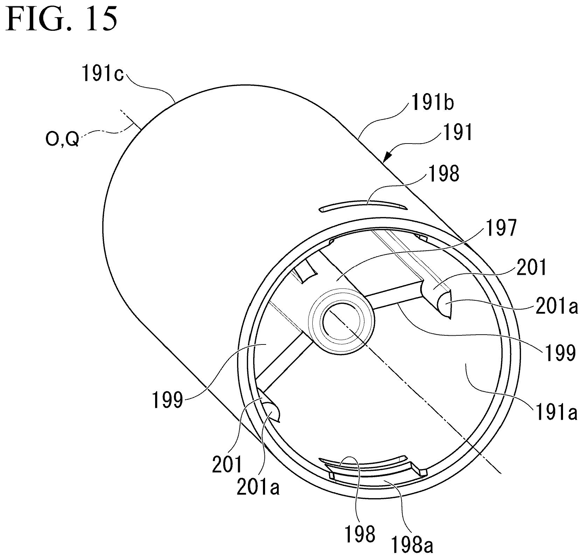

Fig. 15 is a perspective view of the tank 191 viewed from the opening portion 191a side.

As shown in Fig. 13 to Fig. 15, two engagement holes 198 are formed at a slightly bottom portion 191c side more than the opening portion 191a in a circumferential wall 191b of the tank 191. The engagement hole 198 is configured for fixing the heater holder 196 to the tank 191. The engagement hole 198 is formed in a rectangle shape viewed from the radial direction to become long in the circumferential direction. The two engagement holes 198 are disposed to be opposite to each other and to sandwich an axis Q of the tank 191 at two side of the axis Q. The axis Q coincides with the axis O of the main body unit 10 in a state in which the cartridge 11 is accommodated in the transmission cylinder 121. The axis Q is the common axis of each portion configuring the cartridge 11. Hereinafter, the axis Q is not only described as the axis Q of the tank 191, but also used in the description of each portion configuring the cartridge 11.

A guide concave portion 198a is formed on an internal circumferential surface slightly close to the opening portion 191a from the engagement hole 198 in the circumferential wall 191b of the tank 191. The guide concave portion 198a also opens at the opening portion 191a side. The guide concave portion 198a functions to guide an engagement piece 206 described below when fixing the heater holder 196 to the tank 191.

In the bottom portion 191c of the tank 191, a penetration hole 191d penetrating the bottom portion 191c at the center in the radial direction is formed. A flow passage tube 197 is formed in an annular shape and integrally formed in a circumferential edge of the penetration hole 191d to protrude from the internal surface of the bottom portion 191c to the inside of the tank 191. Accordingly, the inside of the flow passage tube 197 and the penetration hole 191d are communicated with each other. The flow passage tube 197 is a flow passage of the atomized aerosol. The flow passage tube 197 extends in a space from the bottom portion 191c to a position slightly close to the opening portion 191a with respect to a substantially center in the axial direction of the tank 191.

Between the internal circumferential surface of the circumferential wall 191b and an external circumferential surface of the flow passage tube 197, a plurality of ribs 199 (three according to the present embodiment) across the circumferential surface 191b and the flow passage 197 are integrally formed. The plurality of ribs 199 are disposed at equal intervals in the circumferential direction so as to be in a radial pattern viewed from the axial direction. The plurality of ribs 199 extend in a space from the bottom portion 191c of the tank 191 to a position slightly in front of an end portion (tip end) at the opening portion 191a side of the flow passage tube 197. The plurality of ribs 199 are configured to support the flow passage tube 197.

In the internal circumferential surface of the circumferential wall 191b, a convex portion 201 is integrally formed at the position where the ribs 199 are formed. The convex portion 201 extends along the ribs 199 in the axial direction. The convex portion 201 is formed in a space from the bottom portion 191c of the tank 191 to a position between an end portion (tip end) at the opening portion 191a side of the rib 199 and a tip end of the flow passage tube 197. The convex portion 201 functions to enhance a mechanical strength of the tank 191 while performing position alignment of the gasket 192.

The gasket 192 is formed to have an external diameter substantially the same as the internal diameter of the tank 191. The gasket 192 is configured to perform position alignment of a mesh body 193 described below while maintaining an orientation of the mesh body 193. In other words, the gasket 192 supports the mesh body 193 described below. An insertion hole 192a capable of being inserted by the flow passage tube 197 is formed in a center in the radial direction of the gasket 192. The gasket 192 is accommodated in the tank 191 such that the flow passage tube 197 is inserted into the insertion hole 192a. A surface 192b is abutted by the end surface 201a of the convex portion 201 such that position alignment of the gasket 192 in the tank 191 is performed. In the state in which the position alignment of the gasket 192 is performed, an external circumferential surface of the gasket 192 comes in contact with the internal circumferential surface of the tank 191. The insertion hole 192a of the gasket 192 comes in contact with the external circumferential surface of the flow passage tube 197.

A plurality of opening portions 192c (four according to the present embodiment) are formed in a major portion between the insertion hole 192a and the external circumferential surface of the gasket 192. The opening portion 192c is formed in an arc shape viewed from the axial direction. The plurality of opening portions 192c are formed by equal intervals in the circumferential direction. Two side sandwiching the gasket 192 in the tank 191 are communicated with each other via the opening portion 192c. The mesh body 193 is disposed on another surface 192d at the opposite side of the surface 192b of the gasket 192.

The mesh body 193 is a porous member having liquid absorbency. The mesh body 193 is formed from a cotton type fibrous material, for example. The mesh body 193 and the gasket 192 are formed in almost the same shape. In other words, the mesh body 193 is formed to have an external diameter substantially the same as the internal diameter of the tank 191. An insertion hole 193a into which the flow passage tube 197 is insertable is formed in a center in the radial direction of the mesh body 193. The flow passage tube 197 is inserted into the insertion hole 193a and the surface 193b of the mesh body 193 overlaps the other surface 192d of the gasket 192 such that positioning of the mesh body 193 is performed. An external circumferential surface of the mesh body 193 comes in contact with the internal circumferential surface of the tank 191. The insertion hole 193a of the mesh body 193 comes in contact with the external circumferential surface of the flow passage tube 197.

The inside of the tank 191 is partitioned into a liquid storage room 202 at the bottom portion 191c side and an opening room 203 at the opening portion 191a side by the mesh body 193. The liquid aerosol source is stored in the liquid storage room 202. The opening room 203 is a room for atomizing the aerosol source absorbed by the mesh body 193.

The other surface 193c at the opposite side of the surface 193b of the mesh body 193 is exposed to the opening room 203. The heater 194 is disposed so as to be connected to the other surface 193c of the mesh body 193 exposed to the opening room 203.

The heater 194 is a configuration for atomizing the liquid aerosol source. The heater 194 is accommodated in the opening room 203. The heater 194 has a wick 204 formed in a substantial U shape, and an electrical heating wire 205 for heating the wick 204. The wick 204 is a porous member formed in a substantial cylindrical shape and having liquid absorbency. The wick 204 is bent and deformed to a substantial U shape.

More specifically, the wick 204 is configured by two axial-direction extending portions 204a extending in the axial direction and a radial-direction extending portion 204c by connecting two end portions of the two axial-direction extending portions 204a via a bending portion 204b. The other end of the axial-direction extending portion 204a is connected to the mesh body 193. Accordingly, the aerosol source absorbed by the mesh body 193 is suctioned by the wick 204.

The electrical heating wire 205 has an electrical heating wire main body 205a formed in a helical shape to surround the circumference of the radial-direction extending portion 204c of the wick 204, and two terminal portions 205b extending from two terminals of the electrical heating wire main body 205a toward the heater holder 196 side along the axial direction. When the wick 204 is heated by the electrical heating wire 205, the aerosol source absorbed by the wick 204 is atomized. Tip ends of the two terminal portions 205b are turned back toward the mesh body 193 side. The two terminal portions 205b are connected to the heater holder 196.

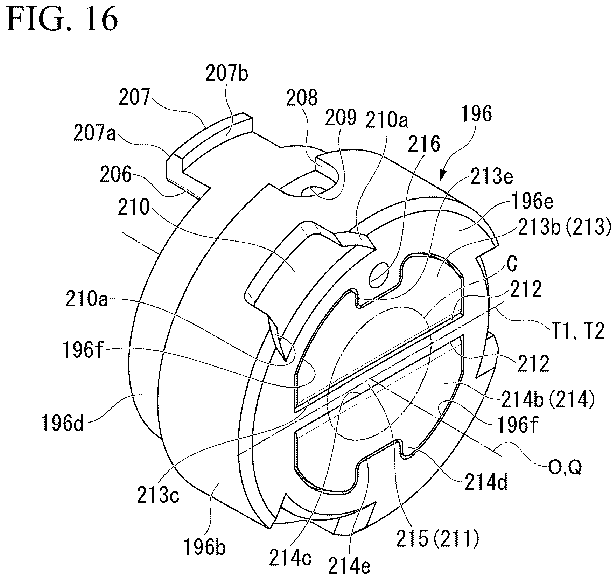

Fig. 16 is a perspective view showing the heater holder 196 viewed from the power unit 21 side (first side in the axial direction).

As shown in Fig . 13 and Fig. 16, the heater holder 196 is formed in a substantial bottomed cylindrical shape. In other words, the heater holder 196 has a cylindrical portion 196b and a bottom portion 196e integrally formed with an end of the cylindrical portion 196b at the opposite side of the tank 191. An opening portion 196a of the heater holder 196 is directed to the tank 191 side and the opening portion 191a of the tank 191 is blocked.

A cylindrical portion 196b of the heater holder 196 is formed to have an external diameter substantially the same as the external diameter of the circumferential wall 191b of the tank 191. A fitting portion 196d whose diameter is reduced via a step surface 196c is formed in a space between a substantial center and the opening portion 196 in the external circumferential surface of the cylindrical portion 196b. The fitting portion 196d is fitted into the internal circumferential surface of the circumferential wall 191b in the tank 191. An end portion at the opening portion 191a side in the circumferential wall 191b of the tank 191 is in contact with the step surface 196c of the cylindrical portion 196b. Accordingly, position alignment of the heater holder 196 with respect to the tank 191 in the axial direction is performed.

Two engagement pieces 206 are integrally formed at positions corresponding to the two engagement holes 198 of the tank 191 in an end portion at the opening portion 196a side of the fitting portion 196d. The two engagement pieces 206 protrude toward the corresponding engagement holes 198. In other words, the two engagement pieces 206 are disposed to be opposite to each other at two side of the axis Q of the heater holder 196 to sandwich the axis Q.

The engagement pieces 206 is engaged with the engagement holes 198 of the tank 191 so as to integrate the tank 191 with the heater holder 196. The engagement pieces 206 are formed to be elastically deformable in the radial direction. An engagement claw 207 insertable into the engagement hole 198 of the tank 191 is formed at a tip end of the engagement piece 206 to protrude outwardly in the radial direction.

The engagement claw 207 is formed to have a triangle cross-sectional shape corresponding to a planar surface defined by the axial direction and the radial direction. In other words, the engagement claw 207 has a surface at a tip end side formed as an inclined surface 207a that is inclined toward the base end side (the fitting portion 196d side) towards the outward in the radial direction. On the other hand, a flat surface 207b at the base end side of the engagement claw 207 is orthogonal with the axial direction.

A concave portion 208 arranged in the axial direction with the engagement claw 207 is formed in part of an external circumferential surface apart from the fitting portion 196d in the cylindrical portion 196b of the heater holder 196. The concave portion 208 opens toward the outward in the radial direction and the step surface 196c side. A first air-suction hole 209 penetrating the cylindrical portion 196b in the thickness direction is formed in the concave portion 208. The inside and the outside of the cylindrical portion 196b are communicated via the first air-suction hole 209.

Furthermore, three engagement concave portions 210 are formed at a bottom portion 196e side in the cylindrical portion 196b of the heater holder 196. The three engagement concave portions 210 are disposed by equal intervals in the circumferential direction (by 120-degree intervals in the circumferential direction) and at positions apart from the positions where the concave portion 208 is formed. The engagement concave portions 210 are formed to open toward the outward in the radial direction and the bottom portion 196e side. A tapered flattening portion 210a is formed at the bottom portion 196e side of the engagement concave portion 210 such that the width of the engagement concave portion 210 in the circumferential direction gradually becomes wider towards the bottom portion 196e.

The vertical engagement convex portions (convex portions) 101a-101c of the first connection member 81 are inserted into the three engagement concave portions 210 respectively. Accordingly, the heater holder 196 (cartridge 11) is connected with the first connection member 81 while position alignment of the heater holder 196 (cartridge 11) and the first connection member 81 in the circumferential direction is performed.

In the bottom portion 196e of the heater holder 196, a connection wall 211 is integrally formed in a substantial plate shape standing from the internal surface in the axial direction. The connection wall 211 extends along the radial direction through the axis Q of the heater holder 196, and two ends in the longitudinal direction of the radial direction are connected to the internal surface of the cylindrical portion 196b. The inside of the heater holder 196 is partitioned into two rooms by such connection wall 211.

Furthermore, in the bottom portion 196e of the heater holder 196, two slits 212 are formed. The two slits 212 are disposed on two surfaces in the plate-thickness direction of the connection wall 211.

Electrodes 213, 214 are disposed on the two surfaces in the plate-thickness direction of the connection wall 211 respectively. The electrodes 213, 214 have extraction electrodes 213a, 214a disposed on the connection wall 211 and connection electrodes (first planar electrode and second planar electrode) 213b, 214b extending in a bending manner from the extraction electrodes 213a, 214a to the external surface of the bottom portion 196e via the corresponding slits 212 respectively. Two terminal portions 205b of the electrical heating wires 205 configuring the heater 194 are connected to the extraction electrodes 213a, 214a respectively.

The connection electrodes 213b, 214b are formed in a substantially semicircular shape at two sides in the radial direction to sandwich an insulation portion 215 described below. More specifically, the two connection electrodes 213b, 214b are disposed to cause sides 213c, 214c in a linear shape when viewed from the axial direction to face each other in the radial direction. Two connection electrodes 213b, 214b are disposed to cause arc-shaped sides 213d, 214d in an arc shape when viewed from the axial direction to configure an external circumferential portion. An end portion of the connection wall 211 is interposed between the sides 213c, 214c of the two connection electrodes 213b, 214b. A tip end of the pin electrode (electrode main body) 49 held by each electrode retainer 50 is in contact with each of the connection electrodes 213b, 214b in a state in which the heater holder 196 (cartridge 11) is connected with the first connection member 81. In other words, the bottom portion 196e of the heater holder 196 functions as an electrode configuration surface being opposite to the base surface 91a in the axial direction in a state in which the cartridge 11 is attached to the main body unit 10.

Each of the connection electrodes 213b, 214b is at least formed on a rotation locus of the pin electrode 49 (first pin electrode 49a and second pin electrode 49b) in a case when the power unit 21 and the cartridge 11 are relatively rotated around the axis O (axis Q). In other words, each of the connection electrodes 213b, 214b is formed in a region including both of a first virtual circle C1 with the axis O as a center and through the first pin electrode 49a, and a second virtual circle C2 with the axis O as a center and through the second pin electrode 49b. According to the present embodiment, the pin electrodes 49a, 49b are disposed in a line symmetry manner such that the virtual circles C1, C2 are coincided with each other.

The end portion of the connection wall 211 interposed between the sides 213c, 214c of the two connection electrodes 213b, 214b extends along the radial direction through the axis Q of the heater holder 196; in other words, the connection wall 211 is disposed on a virtual straight line T1 in a predetermined direction among the virtual straight lines T1 connecting two pin electrodes 49. The predetermined direction is coincided with a virtual straight line T2 through a center in the circumferential direction of one engagement concave portion 210 among the three engagement concave portions 210 formed in the heater holder 196 and the axis Q of the heater holder 196. The connection wall 211 is formed with a width in a short direction (circumferential direction around the axis Q) that is a little larger than a diameter of each pin electrode 49.

The end portion of the connection wall 211 is disposed in this way to function as the insulation portion 215 partitioning the connection electrodes 213b, 214b in the circumferential direction. By disposing the insulation portion 215 on the virtual straight line T2 through the center in the circumferential direction of one engagement concave portion 210 and the axis Q of the heater holder 196, the two connection electrodes 213b, 214b are definitely in contact with the tip ends of each pin electrode 49 respectively in the state in which the heater holder 196 (cartridge 11) and the first connection member 81 are connected with each other. In other words, there is no possibility for either of the two connection electrodes 213b, 214b to come in contact with the two pin electrodes 49 simultaneously. In this manner, the connection electrodes 213b, 214b are formed in a semicircular shape at two sides of the radial direction to sandwich the virtual straight line T2 (insulation portion 215) and include the virtual circles C 1, C1, and expand outwardly (arc-shaped sides 213d, 214d) and inwardly (sides 213c, 214c) in the radial direction.

Concave portions 213e, 214e recessed inwardly in the radial direction are formed in a substantial center in the circumferential direction in the arc-shaped sides 213d, 214d of the two connection electrodes 213b, 214b. In the bottom portion 196e of the heater holder 196, a second air-suction hole 216 penetrating the bottom portion 196e in the thickness direction is formed at a position corresponding to one concave portion 213e between the positions corresponding to the concave portions 213e, 214e of the connection electrodes 213b, 214b. The inside and the outside of the bottom portion 196e are communicated via the second air-suction hole 216.

A concave portion 196f having the same shape with the connection electrodes 213b, 214b viewed from the axial direction is formed at the position corresponding to the connection electrodes 213b, 214b in the bottom portion 196e. The connection electrodes 213b, 214b are accommodated in the concave portion 196f. By forming the concave portion 196f, surfaces of the connection electrodes 213b, 214b and a surface of a portion of the bottom portion 196e where the connection electrodes 213b, 214b are not disposed are positioned on the same plane. A portion of the atomization container 195 is accommodated so as to be fitted in the internal circumferential surface of the cylindrical portion 196b in the heater holder 196.

As shown in Fig. 11, the external circumferential portion of the bottom portion 196e comes in contact with the surrounding convex portion 93 in the axial direction in the state in which the cartridge 11 is attached in the retention unit 22. Accordingly, a space surrounded by the bottom portion 196e and the connection cap 80 (the base surface 91a and the surrounding convex portion 93) forms a buffer space S3 communicating the communication port 51 and the second air-suction hole 216. In the example shown in Fig. 11, the communication port 51 and the second air-suction hole 216 are separate from each other in the axial direction and disposed at positions departing from each other in the circumferential direction. The communication port 51 and the second air-suction hole 216 may be disposed at positions departing from each other in the radial direction.

The communication port 51 according to the present embodiment is communicated with the inside of the flow passage tube 197 via the buffer space S3 and the second air-suction hole 216. A portion of the bottom portion (second surface) 196e in contact with the surrounding convex portion 93 is formed in a flat surface orthogonal with the axial direction. The portion of the bottom portion 196e in contact with the surrounding convex portion 93 may be a convex surface, a concave surface, an inclined surface or the like.

According to the present embodiment, the surrounding convex portion 93 is in close contact with the bottom portion 196e in an elastically deformation state since the cartridge 11 is pressed by the mouthpiece 23. However, the surrounding concave portion 93 and the bottom portion 196e do not have to be in close contact with each other and may be separated from each other. In other words, if it is possible to generate a negative pressure in the pressure change room S1 via the communication port 51 during the suction, a micro gap may be generated between the surrounding convex portion 93 and the bottom portion 196e.

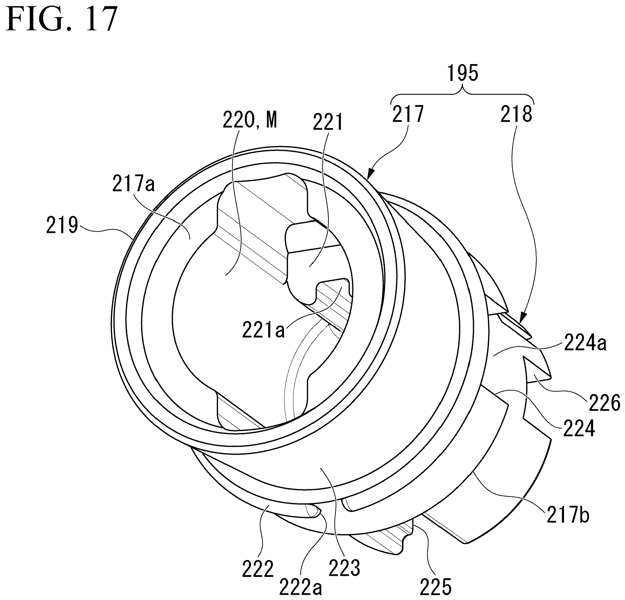

Fig. 17 is a perspective view showing the atomization container 195 viewed from the mesh body 193 side (second side in the axial direction).

The atomization container 195 shown in Fig. 13, Fig. 14, and Fig. 17 is formed from the resin material having the elasticity such as the silicone resin or the like. The atomization container 195 is disposed in a space between the other surface 193c of the mesh body 193 and the vicinity of the bottom portion 196e of the heater holder 196 in the axial direction. In other words, the atomization container 195 is formed in a substantial cylindrical shape so as to surround the circumference of the heater 194, and the atomization container 195 is integrally formed by a cylinder portion 217 fitting to the internal circumferential surface of the circumferential surface 191b in the tank 191 and a fitting portion 218 in a substantial block shape and fitting to the internal circumferential surface of the cylindrical portion 196b in the heater holder 196.

A step surface 217a is formed in a major portion at a center in the radial direction in an end portion at the mesh body 193 side of the cylinder portion 217. By forming the step surface 217a, a protrusion portion 219 in a ring shape is formed that the external circumferential portion of the cylinder portion 217 protrudes toward the liquid retaining boy 193 side. An end portion of the protrusion portion 219 is in contact with the other surface 193c of the mesh body 193. An external diameter of the protrusion portion 219 is substantially the same or a little smaller than the internal diameter of the circumferential wall 191b in the tank 191.

An accommodation concave portion 220 is formed in a major portion of the step surface 217a corresponding to the shape of the heater 194. The accommodation concave portion 220 becomes an atomization room M configured to store the aerosol atomized by the heater 194. The atomization room M is communicated with the flow passage tube 197 of the tank 191.

A bearing surface 221 to which the bending portion 204b of the wick 204 configuring the heater 194 is placed is formed in the accommodation concave portion 220. A concave portion 221a for avoiding interference of the terminal portion 205b of the electrical heating wire 205 configuring the heater 194 is formed in a surface at the internal side in the radial direction of the bearing surface 221.

A seal portion 222 being close to the fitting portion 218 is formed in the external circumferential surface of the cylinder portion 217. The seal portion 222 is formed across the whole circumference except for a notch portion 222a described below and to protrude outwardly in the radial direction. The seal portion 222 functions to secure a sealing performance between the cylinder portion 217 and the circumferential wall 191b of the tank 191, and functions to prevent the atomization container 195 from slipping from the tank 191.

An external diameter of the sealing portion 222 is a little larger than the internal diameter of the circumferential wall 191b of the tank 191. Accordingly, in a state in which the atomization container 195 is accommodated in the tank 191, the seal portion 222 is compressed in the axial direction. Therefore, the sealing performance of the seal portion 222 is secured, and the slipping of the atomization container 195 from the tank 191 is prevented due to the friction resistance of the seal portion 222.

Two notch portions 222a are formed in the seal portion 222. The two notch portions 222a are disposed to be opposite to each other at two sides of the axis Q of the tank 191 to sandwich the axis Q. The external air and a liquid accumulation portion 223 described below are communicated with each other by the notch portions 222a.

The liquid accumulation portion 223 is formed in the external circumferential surface of the cylinder portion 217 between the tip end of the protrusion portion 219 and the seal portion 222. The liquid accumulation portion 223 is configured to once accumulate leaked aerosol source in a case in which the liquid aerosol source stored in the liquid storage room 202 of the tank 191 is leaked via the internal circumferential surface of the circumferential wall 191b of the tank 191 when the mesh body 193 and the wick 204 are saturated.

The liquid accumulation portion 223 is a concave portion configured by obliquely forming the whole external circumferential surface of the cylinder portion 217 such that a gap between the external circumferential surface of the cylinder portion 217 and the circumferential wall 191b of the tank 191 gradually becomes narrower from the seal portion 222 toward the tip end of the protrusion portion 219. In other words, the liquid accumulation portion 223 is the concave portion where the gap between the external circumferential surface of the cylinder portion 217 and the circumferential wall 191b of the tank 191 gradually becomes wider towards the opening portion 191a of the tank 191. Since the liquid accumulation portion 223 is formed in this manner, a narrow portion 279 where a micro gap is generated between the protrusion portion 219 and the circumferential wall 191b of the tank 191 is formed in the vicinity of the protrusion portion 219 of the cylinder portion 217.

The end portion of the protrusion portion 219 in the cylinder portion 217 is in contact with the other surface 193c of the mesh body 193. Furthermore, the external circumferential surface of the mesh body 193 is in contact with the internal circumferential surface of the tank 191. Accordingly, the narrow portion 279 formed between the protrusion portion 219 of the cylinder portion 217 and the circumferential wall 191b of the tank 191 is covered (blocked) by the external circumferential portion of the mesh body 193.

Furthermore, a concave portion 224 receiving the engagement piece 206 is formed at a position corresponding to the engagement piece 206 at the heater holder 196 side more than the seal portion 222 in the external circumferential surface of the cylinder portion 217. The engagement piece 206 is inserted into the concave portion 224 such that position alignment of the atomization container 195 and the heater holder 196 in the circumferential direction is performed. A bottom surface 224a of the concave portion 224 in the cylinder portion 217 is in contact with the internal surface of the engagement piece 206 at the internal side in the radial direction.

The fitting portion 218 of the atomization container 195 is formed in a substantial cylindrical shape capable of fitting into the internal circumferential surface of the cylindrical portion 196b in the heater holder 196. In other words, the fitting portion 218 is formed that an external diameter is reduced than the external diameter of the cylinder portion 217 via the step portion 217b. A slit 225 being insertable into the connection wall 211 of the heater holder 196 is formed in the fitting portion 218. A slit for electrical heating wire that is not shown in figures and communicates with the slit 225 is formed in the fitting portion 218, and the terminal portion 205b of the electrical heating wire 205 is insertable into the slit for electrical heating wire. By inserting the terminal portion 205b of the electrical heating wire 205 into the slit for electrical heating wire, the terminal portion 205b is held by the atomization container 195. The extraction electrodes 213a, 214a disposed in the connection wall 211 and the terminal portion 205b of the electrical heating wire 205 are connected.

A ventilation passage 226 is formed at a position in the fitting portion 218 corresponding to the first air-suction hole 209 of the heater holder 196 and the second air-suction hole 216. Furthermore, a slit 218a communicating the slit 225 and the ventilation passage 226 with the atomization room M (accommodation concave portion 220) of the cylinder portion 217 is formed in the fitting portion 218. The ventilation passage 226 and the atomization room M (accommodation concave portion 220) of the atomization container 195 are communicated via the slit 218a. Accordingly, the atomization room M (accommodation concave portion 220) is communicated with the first air-suction hole 209 and the second air-suction hole 216 of the heater holder 196 via the ventilation passage 226 and the slit 218a.

### (Overall assembly structure of suction device)

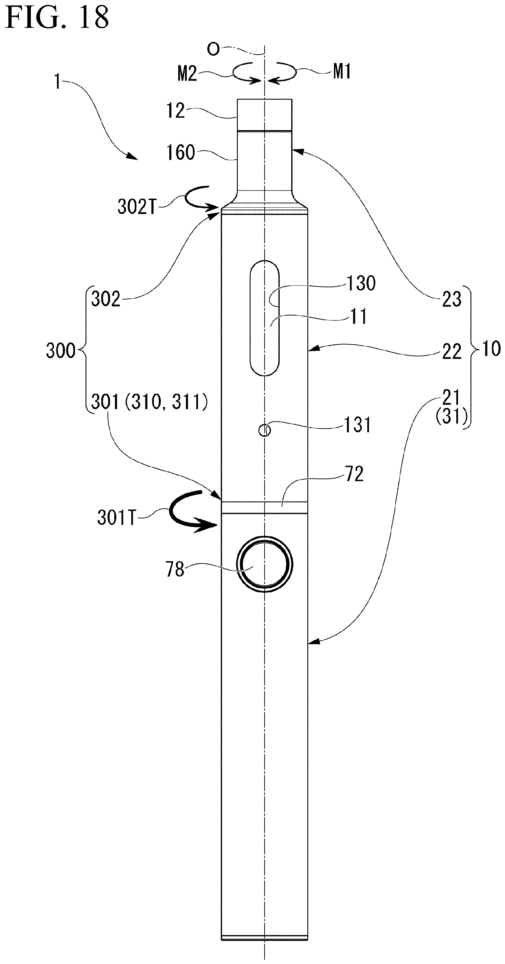

Fig. 18 is a front view of the suction device 1.

As shown in Fig. 18, the main body unit 10 of the suction device 1 has a connection portion 300 configured to connect the power unit 21, the retention unit 22, and the mouthpiece 23 in the axial direction along the axis O (center axis). The connection portion 300 has a first rotation connection portion 301 connecting the power unit 21 and the retention unit 22 and a second rotation connection portion 302 connecting the retention unit 22 and the mouthpiece 23.

In the description hereinafter, in a planar view viewing the power unit 21 side from the mouthpiece 23 side along the axis O, in the circumferential direction around the axis O, a clockwise direction rotating around the axis O is referred to as a rotation direction M1, and a counter-clockwise direction rotating around the axis O is referred to as a rotation direction M2.

The first rotation connection portion 301 is configured to perform a connection and release the connection of the power unit 21 and the retention unit 22 by a relative rotation of the power unit 21 and the retention unit 22 around the axis O. In a case of taking the power unit 21 as a reference, when the retention unit 22 is rotated in the rotation direction M1 with respect to the power unit 21, the power unit 21 and the retention unit 22 are connected. When the retention unit 22 is rotated in the rotation direction M2 with respect to the power unit 21, the connection of the power unit 21 and the retention unit 22 is released.

The first rotation connection portion 301 has a rotation connection mechanism 310 configured by the first connection member 81 and the second connection member 122 shown in Fig. 9, and a lock mechanism 311 configured by the annular piece 82 and the second connection member 122 shown in Fig. 9 and Fig. 10. More specifically, as shown in Fig. 9, the rotation connection mechanism 310 is configured to insert the horizontal engagement convex portion 102 disposed in the first connection member 81 of the power unit 21 into the engagement groove 158 formed in the second connection member 122 of the retention unit 22, and then rotate the retention unit 22 in the rotation direction M1 (see Fig. 18) with respect to the power unit 21 so as to engage the horizontal engagement convex portion 102 to the locking piece 142 and connect the power unit 21 with the retention unit 22.

The lock mechanism 311 is configured to restrict the rotation of the retention unit 22 in the rotation direction M2 for releasing the connection by the rotation connection mechanism 310. More specifically, as shown in Fig. 9 and Fig. 10, the lock mechanism 311 has the bending portion 106 disposed in the annular piece 82 attached to the power unit 21 and protruding outwardly in the radial direction, and a tip end portion 142a disposed in the second connection member 122 of the retention unit 22 and protruding inwardly in the radial direction relatively with respect to a bottom portion of the engagement concave portion 155 in the locking piece 142. The tip end portion 142a of the locking piece 142 is positioned in a movement passage of the bending portion 106 around the axis O.

At the time of the connection in the rotation connection mechanism 310 (when the retention unit 22 is rotated in the rotation direction M1 with respect to the power unit 21), the bending portion 106 and the tip end portion 142a of the locking piece 142 come in contact with each other and the bending portion 106 climbs over the tip end portion 142a while elastically deforming inwardly in the radial direction. The bending portion 106 deforms outwardly in the radial direction after overcoming the tip end portion 142a to restore the shape and engages with the engagement concave portion 155. When the bending portion 106 engages with the engagement concave portion 155, the bending portion 106 and the tip end portion 142a of the locking piece 142 are locked in the rotation direction M1 to be opposite with each other. Accordingly, it is impossible to release the connection of the power unit 21 and the retention unit 22 without applying a certain force.

According to the first rotation connection portion 301, in order to improve manufacturing efficiency or the like, as shown in the present embodiment, even if the power unit 21 and the retention unit 22 are capable of being divided, it is possible to make the connection of the power unit 21 and the retention unit 22 by the rotation connection mechanism 310 easy and improve reliability (connection strength) of the connection state of the power unit 21 and the retention unit 22 by the lock mechanism 311. The locking by the lock mechanism 311 is performed simultaneously with the connection by the rotation connection mechanism 310 such that convenience (usability) of the assembly may be improved.

As shown in Fig. 10, in the lock mechanism 311, the bending portion 106 elastically deforming is disposed at the internal side in the radial direction of the locking piece 142 having a larger thickness and higher rigidness than the annular piece 82. Accordingly, in a state in which the power unit 21 and the retention unit 22 are connected, the bending portion 106 is covered by the locking piece 142 from the external side and protected. Accordingly, even if falling, collision or the like occurs, a number of cases such as the bending portion 106 being damaged become less. Accordingly, strength for repeatedly using the assembly is secured and the reliability of locking is improved.

As shown in Fig. 9, the lock piece 142 configured to lock the bending portion 106 has the engagement groove 158 to which the horizontal engagement convex portion 102 of the rotation connection mechanism 310 is engaged. In this manner, the lock piece 142 forms a portion (engagement groove 158) of the rotation connection mechanism 310 while forms a portion (tip end portion 142a (convex portion)) of the lock mechanism 311 such that it is relatively easy to improve the reliability (connection strength) of the connection state.

As shown in Fig. 18, the second rotation connection member 302 is configured to perform a connection and release the connection between the retention unit 22 and the mouthpiece 23 by the relative rotation of the retention unit 22 and the mouthpiece 23 around the axis O. In a case of taking the retention unit 22 as a reference, when the mouthpiece 23 is rotated in the rotation direction M1 with respect to the retention unit 22, the retention unit 22 and the mouthpiece 23 are connected. When the mouthpiece 23 is rotated in the rotation direction M2 with respect to the retention unit 22, the connection of the retention unit 22 and the mouthpiece 23 is released.

As shown in Fig. 11, the second rotation connection portion 302 has a male screw portion 160a disposed in the mouthpiece 23 and a female screw portion 123a disposed in the retention unit 22. More specifically, the second rotation connection portion 302 is configured to connect the retention unit 22 and the mouthpiece 23 by rotating the male screw portion 160a disposed in the mouthpiece 23 in the rotation direction M1 with respect to the female screw portion 123a disposed in the retention unit 22. The second rotation connection portion 302 is configured to release the connection of the retention unit 22 and the mouthpiece 23 by rotating the male screw portion 160a disposed in the mouthpiece 23 with respect to the female screw portion 123a disposed in the retention unit 22.

As shown in Fig. 18, the rotation direction M1 is a connection direction of the retention unit 22 with respect to the power unit 21 and also a connection direction of the mouthpiece 23 with respect to the retention unit 22. The rotation direction M2 is a connection releasing direction of the retention unit 22 with respect to the power unit 21 and also a connection cancelling direction of the mouthpiece 23 with respect to the retention unit 22. In this manner, the rotation directions for the connection and releasing the connection around the axis O in the first rotation connection portion 301 and the second rotation connection portion 302 are the same as each other. Accordingly, it is possible to provide a unified sense of the assembly operation to the user and improve the convenience (usability).

For a replacement of the cartridge 11 or the like, a frequency of releasing the connection of the mouthpiece 23 and the retention unit 22 is higher than a frequency of releasing the connection of the power unit 21 and the retention unit 22. According to the present embodiment, the connection of the power unit 21 and the retention unit 22 is released by applying a first torque 301T around the axis O in the first rotation connection portion 301, and the connection of the retention unit 22 and the mouthpiece 23 is released in the second rotation connection portion 302 by applying a second torque 302T that is smaller than the first torque 301T. Accordingly, it is possible to prevent co-rotation of the retention unit 22 and the power unit 21 at the time of detaching the mouthpiece 23 from the retention unit 22.

The first torque 301T is a peak value of a torque value when the retention unit 22 is rotated in the rotation direction M2 with respect to the power unit 21, and the first torque 301T depends on a spring modulus or the like corresponding to the elastically deformation in the radial direction of the bending portion 106 as shown in Fig. 9 and Fig. 10. The second torque 302T is a peak value of a torque value when the mouthpiece 23 is rotated in the rotation direction M2 with respect to the retention unit 22, and the second torque 302T depends on a static friction force or the like between the male screw portion 160a and the female screw portion 123a as shown in Fig. 11. It is preferable that the first torque 301T is 1.5 times larger than the second torque 302T, for example.

The first rotation connection portion 301 and the second rotation connection portion 302 are different in connection structure such that it is easy to adjust a magnitude relationship between the first torque 301T and the second torque 302T. For example, if a material selection and a thickness adjustment of the bending portion 106 (annular piece 82) configuring the lock mechanism 311 of the first rotation connection portion 301 is performed, the spring modulus of the bending portion 106 corresponding to the elastically deformation in the radial direction is changed and it is easy to adjust the magnitude of the first torque 301T with respect to the second torque 302T.

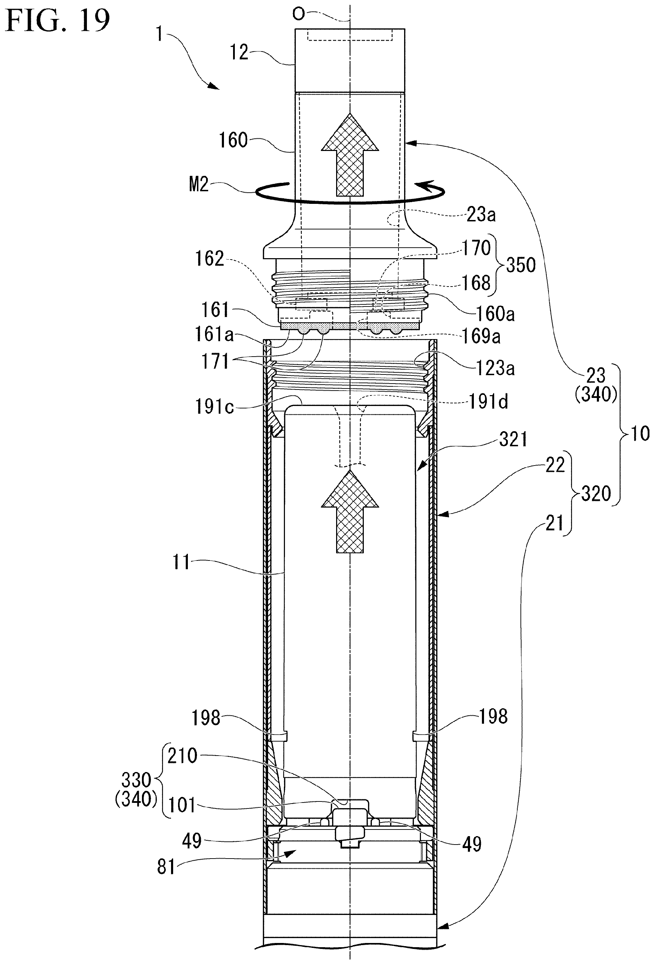

Fig. 19 is a cross-sectional view along the axial direction when the mouthpiece 23 is removed from the suction device 1.

As shown in Fig. 19, in the suction device 1, the cartridge 11 is attachable and detachable in the axial direction by removing the mouthpiece 23 from the main body unit 10. A configuration for removing the mouthpiece 23 from the main body unit 10 is referred to as a cartridge accommodation portion 320. In other words, the cartridge accommodation portion 320 has the retention unit 22 and the power unit 21.

The cartridge accommodation portion 320 forms a cartridge accommodation space 321 in a bottomed cylindrical shape. A circumferential wall of the cartridge accommodation portion 320 forming the cartridge accommodation space 321 is formed by the retention unit 22. A bottom portion of the cartridge accommodation portion 320 forming the cartridge accommodation space 321 is formed by the power unit 21. In other words, the circumferential wall (retention unit 22) of the cartridge accommodation portion 320 is attachable to and detachable from the bottom portion (power unit 21) of the cartridge accommodation portion 320.

The vertical engagement convex portion 101 (the vertical engagement convex portion 101a-10lc are designated to the reference sign 101 after Fig. 19) disposed in the first connection member 81 is formed to stand in the axial direction in the bottom portion of the cartridge accommodation portion 320. The vertical engagement convex portion 101 is disposed to be insertable into the engagement concave portion 210 disposed in the cartridge 11 in the axial direction. In other words, the vertical engagement convex portion 101 and the engagement concave portion 210 are disposed on the same radius with the axis O as a center. The vertical engagement convex portion 101 and the engagement concave portion 210 form a first rotation restriction portion 330 for restricting a relative rotation of the cartridge 11 around the axis O with respect to the cartridge accommodation portion 320 (cartridge accommodation space 321).

In the first rotation restriction portion 330, when the cartridge 11 and the cartridge accommodation portion 320 are relatively rotated around the axis O, the vertical engagement convex portion 101 is inserted into the engagement concave portion 210 disposed on the same radius and the restriction for the rotation of the cartridge 11 around the axis O is performed. Accordingly, position alignment of the cartridge 11 in the circumferential direction is performed, and electrical conduction of the connection electrodes 213b, 214b (see Fig. 10) of the bottom portion 196e of the cartridge 11 and the pin electrode 49 of the power unit 21 is secured.

The first rotation restriction portion 330 together with the mouthpiece 23 configure a position-alignment mechanism 340 for aligning positions of the cartridge 11 with respect to the cartridge accommodation portion 320 by interlocking with screwing of the mouthpiece 23 with respect to the cartridge accommodation portion 320 (retention unit 22). According to the position-alignment mechanism 340, the position alignment of the cartridge 11 may be performed simultaneously with screwing the mouthpiece 23 to the cartridge accommodation portion 320. Accordingly, the position alignment of the cartridge 11 attachable to and detachable from the cartridge accommodation portion 320 becomes easy and complicatedness of the assembling is eliminated. There is not necessity to rotate the cartridge 11 directly by hands.

More specifically, the mouthpiece 23 has the first slip prevention member (cartridge contact portion) 161 for rotating the cartridge 11 around the axis O with respect to the cartridge accommodation portion 320. The first slip prevention member 161 is attached to the mouthpiece main body 160, and the first slip prevention member 161 comes in contact with the cartridge 11 during a period when the mouthpiece main body 160 is connected to the retention unit 22. When the first slip prevention member 161 comes in contact with the cartridge 11, the cartridge 11 begins to rotate with the mouthpiece 23 together, and when a positon of the engagement concave portion 210 in the circumferential direction and a position of the vertical engagement convex portion 101 in the circumferential direction are coincided with each other, the cartridge 11 falls off toward the bottom portion side of the cartridge accommodation portion 320 due to gravity and the vertical engagement convex portion 101 is inserted into the engagement concave portion 210 so as to perform a positioning of the cartridge 11 in the circumferential direction.

Furthermore, when the mouthpiece 23 is screwed, the first slip prevention member 161 is compressed in the axial direction between the cartridge 11 supported by the power unit 21 (the vertical engagement convex portion 101 and the like) and the mouthpiece main body 160. As shown in Fig. 11, the first slip prevention member 161 presses the cartridge 11 toward the power unit 21 in a state in which the mouthpiece 23 is screwed with the retention unit 22. Accordingly, a positioning of the cartridge 11 in the axial direction is performed.

As described above, the first slip prevention member 161 is formed from the silicone resin such that it is easy to cause the friction force for rotating the cartridge 11 in the circumferential direction and a pressing force for pressing the cartridge 11 in the axial direction to be realized. As shown in Fig. 19, the first slip prevention member 161 has the engagement protrusion 171 formed on an opposite surface 161a being opposite to the cartridge 11. According to the engagement protrusion 171, a contact of the first slip prevention member 161 with respect to the cartridge 11 is not a plane contact such that a contact pressure increases and it becomes easy to realize the friction force in the circumferential direction and the pressing force in the axial direction.

As shown in Fig. 11, the engagement protrusion 171 is pressed and crushed in the axial direction such that the penetration hole 191d of the cartridge 11 and the communication hole 169a of the first slip prevention member 161 are airtightly sealed with each other, the flow passages of the cartridge 11 and the mouthpiece 23 are communicated, and the aerosol generated in the cartridge 11 is capable of being suctioned through the mouthpiece 23. The engagement protrusion 171 is formed in a double annular shape (see Fig. 12) such that a double seal having a high airtightness may be formed.

As shown in Fig. 19, the mouthpiece 23 has a second rotation restriction portion 350 for restricting a relative rotation of the first slip prevention member 161 with respect to the mouthpiece main body 160. The second rotation restriction portion 350 is formed by the fitting protrusion 170 (see Fig. 12) disposed in the first slip prevention member 161 and the oval-shaped penetration hole 168 (see Fig. 12) disposed in the mouthpiece main body 160. A pair of the fitting protrusions 170 extend toward the mouthpiece main body 160 in the axial direction and fit with two end portions in the longitudinal direction of the penetration hole 168.

According to the second rotation restriction portion 350, even if condensed aerosol is stored in a space between the mouthpiece main body 160 and the first slip prevention member 161, idling operation (slip) of the first slip prevention member 161 with respect to the mouthpiece main body 160 may be prevented. Accordingly, a positioning of the cartridge 11 in the circumferential direction may be definitely performed. The penetration hole 168 may be formed in the oval shape to be integrally formed with the suction port 23a.

### (Effects)

### (Assembly method of the suction device)

Next, an assembly method of the suction device 1 will be described.

As shown in Fig. 2, in order to assemble the suction device 1 according to the present embodiment, the retention unit 22 is assembled to the power unit 21 at first. More specifically, after inserting the horizontal engagement convex portion 102 into the engagement groove 158 in the axial direction, the power unit 21 and the retention unit 22 are relatively rotated around the axis O. Therefore, the power unit 21 and the retention unit 22 are assembled with each other in the first rotation connection portion 301 in a state in which position alignments in the axial direction and the circumferential direction are performed. At the time of detaching the power unit 21 and the retention unit 22, operations reverse to the above-described operations are performed.

Subsequently, the cartridge 11 is inserted into the retention unit 22. More specifically, the cartridge 11 is inserted into the retention unit 22 in a state in which the connection electrodes 213b, 214b of the cartridge 11 are directed to the retention unit 22 side in the axial direction. In a case in which the positions of the vertical engagement convex portions 101a-101c of the power unit 21 and the position of the engagement concave portion 210 of the cartridge 11 are coincided with each other in the circumferential direction, each of the vertical engagement convex portions 101a-101c is inserted into the corresponding engagement concave portion 210. In the engagement concave portion 210, the flattening portion 210a is formed and on the other hand, inclined surfaces are formed at tip end of the vertical engagement convex portions 101a-101c. Accordingly, the vertical engagement convex portions 101a-101c are smoothly inserted into the engagement concave portion 210. Accordingly, position alignments of the cartridge 11 with respect to the power unit 21 in the circumferential direction and the axial direction are performed and the cartridge 11 is assembled with the power unit 21 at a regular position.

In other words, one pin electrode 49 of the pin electrodes 49 of the power unit 21 and either connection electrode 213b or 214b of the connection electrodes 213b, 214b in the cartridge 11 are connected with each other. The other pin electrode 49 and the other connection electrode 213b or 214b of the connection electrodes 213b, 214b in the cartridge 11 are connected with each other. The power of the power unit 21 is transmittable to the electrical heating wire 205 of the heater 194 via the connection electrodes 213b, 214b (electrodes 213, 214). Furthermore, the buffer space S3 is formed by the cartridge 11 and the connection cap 80 by engaging the bottom portion 196e of the cartridge 11 with the surrounding convex portion 93.

Subsequently, the mouthpiece 23 is assembled with the retention unit 22 by the second rotation connection portion 302. More specifically, the male screw potion 160a of the mouthpiece main body 160 is screwed to the female screw portion 123a of the sleeve 123. Therefore, the first slip prevention member 161 comes in contact with the bottom portion 191c of the cartridge 11. When the mouthpiece 23 is further tightened in this state, the first slip prevention member 161 is elastically deformed such that the cartridge 11 is held in the retaining in the retention unit 22 in a state in which the cartridge 11 is pressed toward the power unit 21 side in the axial direction. A movement of the cartridge 11 with respect to the power unit 21 in the circumferential direction is restricted by the vertical engagement convex portions 101a-101c. Accordingly, the cartridge 11 is configured to not to rotate following the mouthpiece 23 due to the friction force applied between the first slip prevention member 161 and the cartridge 11.

Subsequently, the tobacco capsule 12 is inserted into the mouthpiece 23. More specifically, the tobacco capsule 12 is fitted into the mouthpiece main body 160 in a state of directing the mesh opening toward the mouthpiece 23.

Therefore, the assembly of the suction device 1 is finished.

However, during the insertion of the cartridge 11, there is a case in which the positions of the vertical engagement convex portions 101a-101c of the power unit 21 and the position of the engagement concave portion 210 of the cartridge 11 are not coincided in the circumferential direction due to an orientation of the cartridge in the circumferential direction. In this case, the bottom portion 196e of the cartridge 11 enters a state of climbing on the vertical engagement convex portions 101a-101c (hereinafter simply referred to as a "climb-on state").

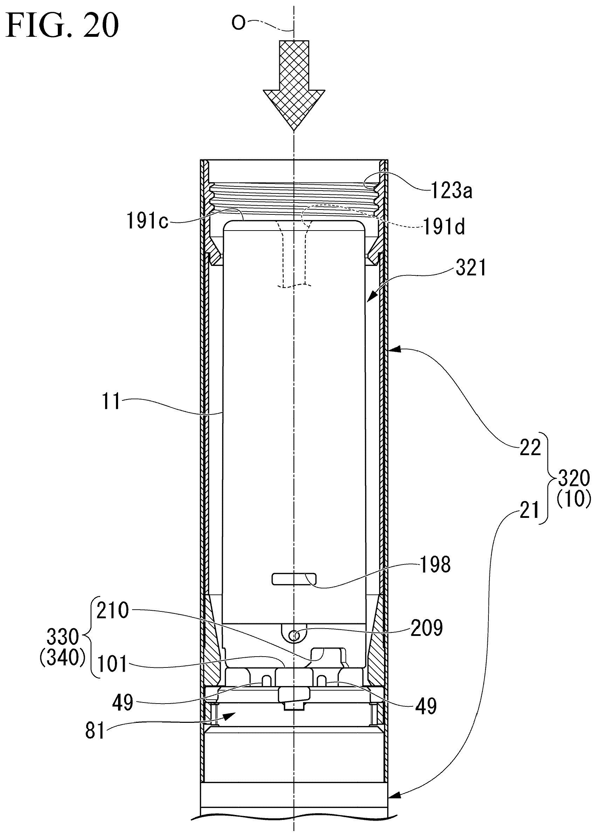

Fig. 20 is a view showing the state in which the cartridge 11 climbs on the vertical engagement convex portion 101.

As shown in Fig. 20, in the climb-on state of the cartridge 11, movement of the cartridge 11 toward the power unit 21 side in the axial direction with respect to the power unit 21 is restricted. Accordingly, the pin electrodes 49 and the connection electrodes 213b, 214b are separated in the axial direction, and a conduction of the power unit 21 and the cartridge 11 is not secured. In the climb-on state, even in a case in which the pin electrodes 49 and the connection electrodes 213b, 214b are in contact, there is a possibility that the pin electrodes 49 and the connection electrodes 213b, 214b are not disposed in desired positions in the circumferential direction.

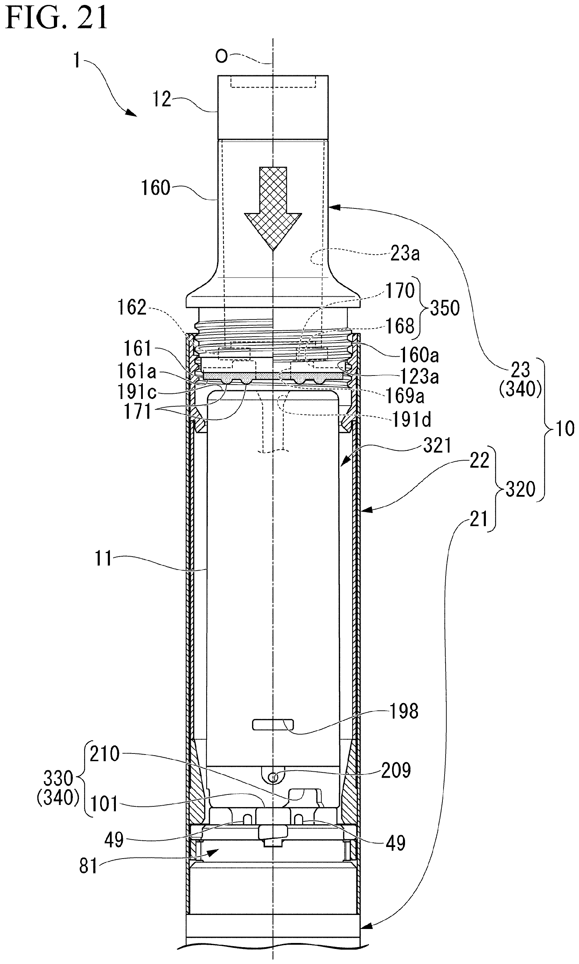

Fig. 21 is a view showing a state of screwing the mouthpiece 23 in the climb-on state of the cartridge 11.

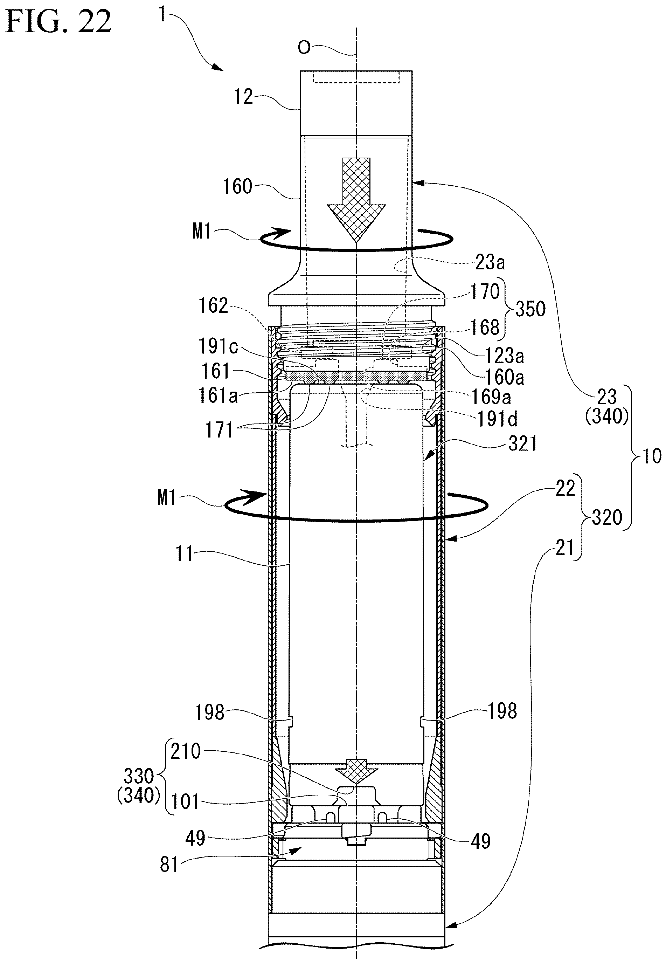

As shown in Fig. 21, when the cartridge 11 is kept in the climb-on state and the mouthpiece 23 is rotated to be screwed with the retention unit 22, as shown in following Fig. 22, the first slip prevention member 161 comes in contact with the cartridge 11 at least before the screwing is finished. More specifically, as shown in Fig. 21, at a moment when the male screw portion 160a of the mouthpiece 23 is about to engage with the female screw portion 123a of the retention unit 22, the first slip prevention member is not in contact with the cartridge 11; however, as shown in Fig. 22, when the male screw portion 160a is screwed with the female screw portion 123a and rotated by a half-rotation or 1, 2 rotations, the first slip prevention member 161 is in contact with the cartridge 11.

Fig. 22 is a view showing a state in which the mouthpiece 23 and the cartridge 11 are rotated together.

As shown in Fig. 22, in the state in which the first slip prevention member 161 is in contact with the cartridge 11, if the screwing operation of the mouthpiece 23 is continued, the mouthpiece 23 and the cartridge 11 are rotated together due to the friction force applied between the first slip prevention member 161 and the cartridge 11. In other words, due to the screwing operation of the mouthpiece 23, the cartridge 11 is pressed toward the power unit 21 side in the axial direction and rotated in the circumferential direction (tightening direction (rotation direction M1)).

Subsequently, when the positions of the connection electrodes 213b, 214b in the circumferential direction and the positions of the vertical engagement convex portions 101a-101c in the circumferential direction are coincided with each other, the vertical engagement convex portions 101a-101c enter the corresponding engagement concave portions 210 respectively. In other words, the cartridge 11 is assembled at the regular position by allowing the movement of the cartridge 11 in the axial direction with respect to the power unit 21. Accordingly, the pin electrodes 49 and the connection electrodes 213b, 214b are in contact in a state in which the movement of the cartridge 11 in the axial direction with respect to the power unit 21 is restricted.

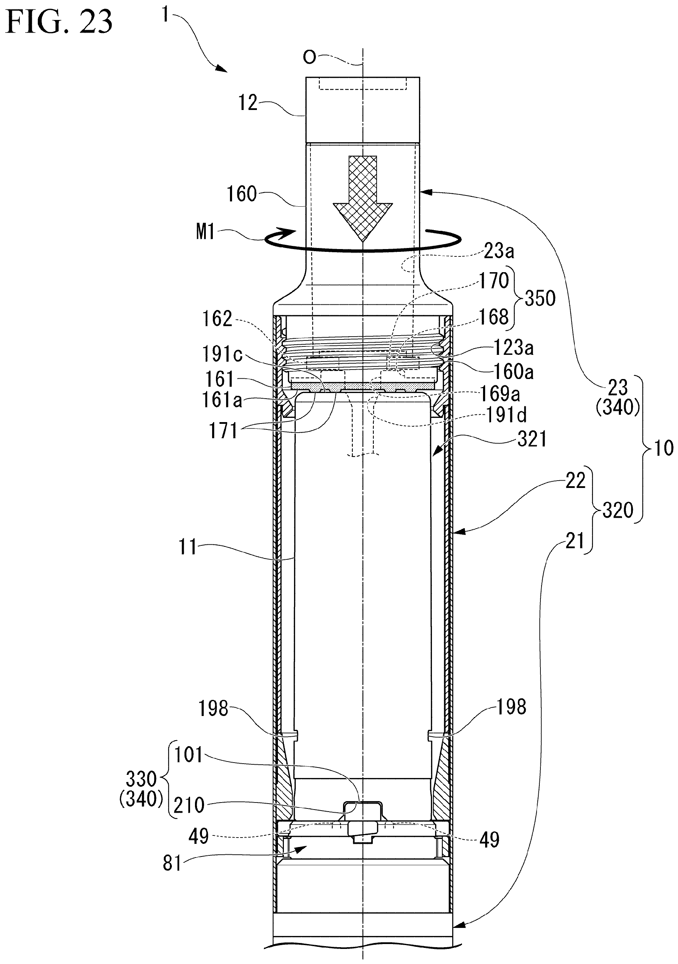

Fig. 23 is a descriptive view showing the state in which the mouthpiece 23 is finally tightened.

As shown in Fig. 23, due to the position alignment of the vertical engagement convex portion 101 and the engagement concave portion 210 in the circumferential direction, when the movement of the cartridge 11 in the axial direction is allowed, the mouthpiece 23 may be further screwed. When the mouthpiece 23 is finally screwed, the connection electrodes 213b, 214b are pressed by the pin electrodes 49 and the first slip prevention member 161 between the cartridge 11 supported by the power unit 21 and the mouthpiece main body 160 is compressed in the axial direction that the positioning of the cartridge 11 in the axial direction is performed. In this manner, the positioning of the cartridge 11 in the circumferential direction and the axial direction and further the electrically conduction of the cartridge 11 and the power unit 21 are performed by the screwing of the mouthpiece 23. Additionally, a gap between the cartridge 11 and the mouthpiece 23 is sealed by the engagement protrusion 171 of the first slip prevention member 161 being compressed in the axial direction.

In this manner, when the cartridge 11 is assembled in the regular position, the surrounding convex portion 93 of the connection cap 80 comes in contact with the cartridge 11. Accordingly, the buffer space S3 (see Fig. 3) whose circumference is surrounded by the surrounding convex portion 93 is formed between the bottom portion 196e of the heater holder 196 of the cartridge 11 and the connection cap 80.

### (Assembly method of cartridge)

Next, an assembly method of the cartridge 11 will be described.

Firstly, the liquid aerosol source is filled in the liquid storage room 202 of the tank 191, and then the gasket 192 and the mesh body 193 are inserted from the opening portion 191a of the tank 191 in this sequence. At this time, the surface 192b of the gasket 192 is in contact with the end surface 201a of the convex portion 201 of the tank 191. The suction-port-side surface 193b of the mesh body 193 is caused to overlap the other surface 192d of the gasket 192. Accordingly, the inside of the tank 191 is correctly partitioned into the liquid storage room 202 and the opening room 203 by the mesh body 193. The mesh body 193 itself is soft; however, the orientation the mesh body 193 is maintained by the gasket 192 and the poisoning thereof is performed by the gasket 192.

The heater 194 and the atomization container 195 are assembled to the heater holder 196 parallely to the above-described process. More specifically, firstly, the heater 194 is assembled to the accommodation concave portion 220 of the atomization container 195. Subsequently, the fitting portion 218 side of the atomization container 195 is directed to the opening portion 196a of the heater holder 196 and the atomization container 195 is inserted into the heater holder 196. The fitting portion 218 is fitted to the internal circumferential surface of the cylindrical portion 196b in the heater holder 196. At this time, directions of the connection wall 211 of the heater holder 196 and the slit 225 of the fitting portion 218 are aligned and the connection wall 211 is inserted into the slit 225.

Subsequently, the heater holder 196 is assembled to the opening portion 191a of the tank 191. More specifically, the engagement piece 206 side of the heater holder 196 is directed to face the opening portion 191a side of the tank 191 and the heater holder 196 is inserted into the opening portion 191a of the tank 191. At this time, positions of the engagement hole 198 and the guide concave portion 198a formed in the circumferential wall 191b of the tank 191 and a position of the engagement piece 206 of the heater holder 196 are aligned.

When the heater holder 196 is inserted into the opening portion 191a of the tank 191 in this state, firstly, the inclined surface 207a formed in the engagement claw 207 of the engagement piece 206 comes in contact with the circumferential wall 191b of the tank 191. The engagement claw 207 smoothly comes in contact with the guide concave portion 198a of the tank 191 by the inclined surface 207a.

Thereafter, when the heater holder 196 is further pushed into the inside of the tank 191, the engagement claw 207 is received and carried in the guide concave portion 198a. The engagement piece 206 is pressed to be elastically deformed inwardly in the radial direction by the guide concave portion 198a. At this time, the engagement piece 206 is smoothly elastically deformed inwardly in the radial direction by the inclined surface 207a of the engagement claw 207. The two engagement pieces 206 are disposed at two sides of the axis Q to sandwich the axis Q and face each other such that it is difficult for forces applied inwardly in the radial direction to the two engagement pieces 206 to be biased when the heater holder 196 is viewed as a whole. At this time, the forces causing the engagement pieces 206 to be elastically deformed are balanced such that it is easy for the heater holder 196 to be inserted into the opening portion 191a of the tank 191. The bottom surface 224a of the concave portion 224 of the atomization container 195 is in contact with the internal surface at the internal side of the engagement piece 206 in the radial direction. Accordingly, when the engagement piece 206 is elastically deformed inwardly in the radial direction, the concave portion 224 of the atomization container 195 is slightly deformed inwardly in the radial direction.

Thereafter, when the heater holder 196 is further pushed, the engagement claw 207 moves along the guide concave portion 198a. Then, the engagement claw 207 climbs on a terminal end of the guide concave portion 198a (end portion at the engagement hole 198 side of the tank 191), and further the engagement claw 207 is inserted into the engagement hole 198 of the tank 191 by a restoring force of the engagement piece 206 and a restoring force of the concave portion 224 of the atomization container 195. Accordingly, the heater holder 196 is fixed to the tank 191 and the assembly of the cartridge 11 is finished.

In a state in which the heater holder 196 is fixed to the tank 191, a surface at the external side in the radial direction of the engagement piece 206 is covered by the circumferential wall 191b of the tank 191. When the engagement of either of the two engagement claws 207 is about to be released, for example, when the tank 191 or the heater holder 196 is about to be tilted so as to cause one of the engagement claw 207 to be removed from the engagement hole 198, the other engagement claw 207 is pressed outwardly in the radial direction. Accordingly, once the engagement hole 198 and the engagement piece 206 are engaged with each other, it is difficult to release the engagement.

### (Usage method of suction device)

When the suction device 1 is used, the user operates to press the button 78. At this time, for example, by pressing the button 78 for several times (for example, five times), a start-up preparation signal is output from the switch element 52 to a controller included in the first substrate module 34.

Subsequently, the user suctions in a state of biting the mouthpiece 23 or the tobacco capsule 12. Therefore, the air in the retention unit 22 is suctioned and tie pressure inside the retention unit 22 becomes negative. When the pressure inside the retention unit 22 becomes negative, the air in the pressure change room S1 is suctioned through the inside of the atomization container 195 (inside the atomization room M) of the cartridge 11, the buffer space S3, and the communication port 51 such that it also becomes the negative pressure inside the pressure change room S 1. More specifically, the air in the pressure change room S1 flows into the buffer space S3 through the communication port 51 and then flows into the heater holder 196 through the second air-suction hole 216. The air flowing into the heater holder 196 passes through the flow passage tube 197 through the ventilation passage 226 and the atomization container 195 and then enters the mouth of the user through the mouthpiece 23. The pressure sensor 53 outputs a start-up signal to the controller when the pressure sensor 53 detects that the pressure inside the pressure change room S1 is less than a predetermined value, for example.

The controller receiving the start-up signal causes the heater 194 of the cartridge 11 to be electrified. Since it becomes the negative pressure inside the retention unit 22, fresh air is introduced in the retention unit 22 through the ventilation hole 131. Furthermore, the fresh air is introduced into the atomization room M of the cartridge 11 (the opening room 203 of the tank 191) through the first air-suction hole 209 formed in the heater holder 196 of the cartridge 11 and the ventilation passage 226 of the atomization container 195.

The electrical heating wire 205 generates heat when the heater 194 is electrified. Therefore, the liquid aerosol source impregnating the wick 204 through the mesh body 193 is heated and atomized. The atomized aerosol fills the atomization room M. Then, the atomized aerosol together with the fresh air introduced into the atomization room M is suctioned to the mouthpiece 23 side through the flow passage tube 197 of the tank 191. Thereafter, a gaseous mixture of the atomized aerosol and air enters the mouth of the user through the tobacco capsule 12. Accordingly, the user may taste a flavor of the tobacco.

### (Effect of the cartridge)

In the cartridge 11, the liquid aerosol source stored in the liquid storage room 202 of the tank 191 is absorbed by the mesh body 193 and further absorbed by the wick 204. When the mesh body 193 and the wick 204 is saturated (exceeding a liquid retention force), there is a risk that the liquid aerosol source leaks out to the heater holder 196 side from an interval between the external circumferential portion of the mesh body 193 and the internal circumferential surface of the circumferential wall 191b in the tank 191 and through the internal circumferential surface.

The liquid accumulation portion 223 is formed on the external circumferential surface of the atomization container 195 positioned at the heater holder 196 side of the mesh body 193. Accordingly, the liquid aerosol source is stored in the liquid accumulation portion 223 and leakage to the heater holder 196 side is prevented.

More specifically, according to the present embodiment, a volume (space volume) of the liquid accumulation portion 223 is approximately 53.4 cubic millimeters. In a case of assuming that a residual liquid quantity in the liquid storage room 202 of the tank 191 is 1/3 and a headspace volume expansion coefficient (a volume expansion coefficient of the air in the residual 2/3 space in the liquid storage room 202) is 6%, there is approximately 100 cubic millimeters of the liquid aerosol source being extruded from the liquid storage room 202 due to the air expansion in the liquid storage room 202 of the tank 191. Among the extruded liquid aerosol source, there is approximately 20-30 cubic millimeters of the liquid aerosol source may be retained by the mesh body 193 and the wick 204. Among the approximately 100 cubic millimeters of the liquid aerosol source, the remaining 70-80 cubic millimeters of the liquid aerosol source is accumulated in the accumulation portion 223.

The liquid accumulation portion 223 is formed that the gap between the external circumferential surface of the cylinder portion 217 and the circumferential wall 191b of the tank 191 gradually becomes narrower towards the tip end of the protrusion portion 219 from the seal portion 222. In other words, in the vicinity of the protrusion portion 219 of the cylinder portion 217, the narrow portion 279 where the gap between the protrusion portion 219 and the circumferential wall 191b of the tank 191 becomes narrow is formed. Accordingly, among the liquid aerosol source extruded from the liquid storage room 202 of the tank 191, it is easy for the residual aerosol source after the mesh body 193 and the wick 204 are saturated to be suctioned due to the narrow portion 279 and the residual aerosol source actively flows to the liquid accumulation portion 223 through the narrow portion 279.

In other words, the liquid aerosol source stored in the liquid storage room 202 of the tank 191 is firstly absorbed by the mesh body 193 and then absorbed by the wick 204. After the mesh body 193 and the wick 204 are saturated, the liquid aerosol source is suctioned by the narrow portion 279 and accumulated in the liquid accumulation portion 223.

On the other hand, when the saturation state of the mesh body 193 is resolved, the liquid aerosol source stored in the liquid accumulation portion 223 is suctioned through the narrow portion 279 (the interval between the protrusion portion 219 and the circumferential wall 191b of the tank 191). Then, the liquid aerosol source is absorbed by the mesh body 193. In other words, the liquid aerosol source stored in the liquid accumulation portion 223 flows back to the liquid storage room 202 of the tank 191 through the narrow portion 279. At this time, since the narrow portion 279 is covered (blocked) by the external circumferential portion of the mesh body 193, a capillary force due to the mesh body 193 also applies such that the liquid aerosol source efficiently flows back to the liquid storage room 202 of the tank 191.

Since two notch portions 222a are formed in the seal portion 222 of the cylinder portion 217, the liquid accumulation portion 223 and the external air are communicated through the notch portions 222a of the seal portion 222 and a gap between the engagement hole 198 of the tank 191 and the engagement piece 206 (engagement claw 207) of the heater holder 196. As another example, the liquid accumulation portion 223 and the external air may be communicated through the notch portions 222a of the seal portion 222 and the first air-suction hole 209 of the heater holder 196. Accordingly, it is impossible to generate a pressure difference between the inside and outside the liquid accumulation portion 223. As a result, an unintentional leakage of the liquid aerosol source to the outside from the liquid accumulation portion 223 is prevented and the liquid aerosol source efficiently flows back to the liquid storage room 202 of the tank 191.

### (Effect)

In this manner, in the cartridge 11 according to the present embodiment, the fitting portion 196d is formed in the cylindrical portion 196b of the heater holder 196. The fitting portion 196d is fitted to the circumferential wall 191b of the tank 191. As being configured in this manner, two engagement holes 198 are formed in the circumferential wall 191b of the tank 191, and two engagement pieces 206 being engaged with the two engagement holes 198 respectively are formed in the fitting portion 196d of the heater holder 196. The engagement piece 206 is elastically deformable in the radial direction. Accordingly, in a state in which the heater holder 196 is fitted and fixed to the tank 191, a surface of the engagement piece 206 at the external side in the radial direction is covered by the circumferential wall 191b of the tank 191 such that the surface is impossible to be touched by the engagement piece 206. In other words, after fitting and fixing the heater holder 196 to the tank 191, it is impossible to intentionally cause the engagement piece 206 to elastically deform. Accordingly, it is possible to make it difficult to intentionally disassemble the tank 191 and the heater holder 196.

There is no necessity to form the groove portion over the whole circumference of the tank 191 and the heater holder 196 as in the past to make it difficult to disassemble the tank 191 and the heater holder 196. Accordingly, it is possible to secure efficient mechanical strength of the tank 191 and the heater holder 196.

The engagement of the tank 191 and the heater holder 196 is configured by the engagement holes 198 and the engagement claws 207 of the engagement pieces 206. According to such a simple structure, the tank 191 and the heater holder 196 may be definitely engaged. It is possible to make it difficult to cause the tank 191 to be attached to or detached from the heater holder 196 that are once fixed.

The engagement claw 207 is formed as the inclined surface 207a that a surface at the tip end side is inclined toward the base end side (the fitting portion 196d side) towards the outward in the radial direction. On the other hand, the flat surface 207b at the base end side of the engagement claw 207 is orthogonal to the axial direction. Accordingly, when the heater holder 196 is inserted into the opening portion 191a of the tank 191, it is possible to cause the engagement claw 297 smoothly come in contact with the circumferential wall 191b of the tank 191 by the inclined surface 207a. Accordingly, the engagement claw 207 may be smoothly inserted into the engagement hole 198.

On the other hand, after inserting the engagement claw 207 into the engagement hole 198, since the flat surface 207b at the base end side of the engagement claw 207 is in contact with the internal circumferential edge of the engagement hole 198, even if the heater holder 196 is about to be pulled from the tank 191, it is difficult for the pulling force to be dispersed in the radial direction. In other words, it is difficult to cause the engagement piece 206 to be elastically deformed. Accordingly, it is possible to make the engagement of the engagement hole 198 and the engagement claw 207 more difficult to be released.

The two engagement holes 198 and the two engagement pieces 206 are disposed at two sides to sandwich the axis O, Q. Accordingly, when viewing the whole heater holder 196, it is difficult for a force inwardly in the radial direction that is applied to the two engagement pieces 206 to be biased. Accordingly, the force applied for making the engagement pieces 206 to elastically deform is balanced and it is easy for the heater holder 196 to be inserted into the opening portion 191a of the tank 191.

When the tank 191 or the heater holder 196 is inclined for pulling the engagement claw 207 from the engagement hole 198 so as to release either of the engagement of the two engagement claws 207, the engagement claw 207 at the other side is pressed outwardly in the radial direction. Accordingly, it is possible to cause the engagement hole 198 and the engagement piece 206 that are once fixed more difficult to be disassembled.

The guide concave portion 198a is formed in the circumferential wall 191b of the tank 191. When the heater holder 196 is inserted into the tank 191, the engagement claw 207 is received and accommodated in the guide concave portion 198a. In this manner, it is possible to perform the position alignment of the tank 191 and the heater holder 196 (cylindrical portion 196b) while performing the fitting of the tank 191 and the heater holder 196 easily by using the guide concave portion 198a.

The fitting portion 218 of the atomization container 195 is fitted to the internal circumferential surface of the cylindrical portion 196b of the heater holder 196. The bottom surface 224a of the concave portion 224 of the atomization container 195 is in contact with an internal surface of the engagement piece 206 inwardly in the radial direction. Accordingly, the elastic force of the atomization container 195 may apply to the engagement piece 206. In other words, when the engagement piece 206 is about to be elastically deformed inwardly in the radial direction, an elastic force outwardly in the radial direction applies to the engagement piece 206 by the elastic force of the atomization container 195. In other words, the atomization container 195 (the fitting portion 218) functions as an elastic supplying member configured to supply an elastic force for biasing the engagement piece 206 outwardly in the radial direction. Accordingly, it is possible to cause the engagement hole 198 and the engagement piece 206 that are once fixed more difficult to be disassembled.

According to the above-described embodiment, a case in which the liquid accumulation portion 223 is the concave portion configured by obliquely forming the overall external circumferential surface so as to make the gap between the external circumferential surface of the cylinder portion 217 and the circumferential wall 191b of the tank 191 to gradually become narrower towards the tip end of the protrusion portion 219 from the seal portion 222 is described; however, the liquid accumulation portion 223 is not limited to the configuration. The liquid accumulation portion 223 only has to be a configuration capable of storing the liquid aerosol source. For example, a configuration may be configured by forming the overall external circumferential surface in a bending manner so as to make the gap between the external circumferential surface of the cylinder portion 217 and the circumferential wall 191b of the tank 191 to gradually become narrower towards the tip end of the protrusion portion 219 from the seal portion 222. Simply, the liquid accumulation portion 223 only has to be a concave portion communicating with the narrow portion 279 and the shape is not limited. Furthermore, as shown in each modification example described below, a cylinder portion 217 having the accumulation portion 223 may be provided.

### (First modification example)

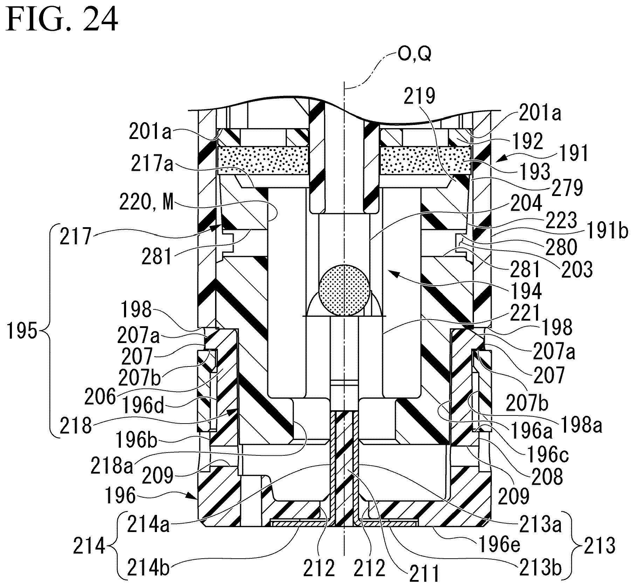

Next, a first modification example of the above-described embodiment will be described with reference to Fig. 24.

Fig. 24 is an enlarged cross-sectional view in the axial direction showing a portion corresponding to the atomization container 195 of the cartridge 11 according to the first modification example. Fig. 24 corresponds to Fig. 13.

As shown in Fig. 24, the first modification example is different from the embodiment in that a shape of the atomization container 195 is different.

According to the first modification example, a concave portion 280 having a cross section in a substantial rectangle shape is formed across the whole circumference in the liquid accumulation portion 223 in the cylinder portion 217 of the atomization container 195. Two penetration holes 281 are formed in the concave portion 230 so as to allow communication between the inside and outside of the cylinder portion 217, in other words, communicate the liquid accumulation portion 223 and the accommodation concave portion 220 (atomization room M). The seal portion 222 and the notch portion 222a (see Fig. 17) are not formed in the atomization container 195.

The two penetration holes 281 are formed along a direction orthogonal to the axis Q. The two penetration holes 281 are formed to be opposite to each other with the axis Q as a center. In this manner, the penetration holes 281 communicate the first air-suction hole 209 of the heater holder 196 and the liquid accumulation portion 223 instead of the notch portion 222a. In other words, the first air-suction hole 209 of the heater holder 196 and the liquid accumulation portion 223 are communicated with each other through the penetration holes 281, the accommodation concave portion 220, and the slit 218a of the atomization container 195.

A hole diameter of the penetration hole 281 is set to be suitable for a surface tension of the liquid aerosol source to apply. Accordingly, even in a case the liquid aerosol source is stored in the liquid accumulation portion 223, it is impossible that the liquid aerosol source flows out to the accommodation concave portion 220 side through the penetration hole 281.

In this manner, according to the cartridge 11 of the first modification example, the same effect may be achieved with the above-described embodiment. In addition, the volume of the liquid accumulation portion 223 may be increased by forming the concave portion 230 in the cylinder portion 217. Since the penetration holes 281 are formed in the concave portion 280, it is possible to prevent a generation of a pressure difference between the inside and outside the liquid accumulation portion 223 without forming the notch portion 222a in the seal portion 222.

According to the first modification example, a case in which the concave portion 230 formed in the cylinder portion 217 has the cross section in the substantial rectangle shape is described. However, it is not limited thereto, the concave portion 230 only has to be formed across the whole circumference of the cylinder portion 217. For example, the concave portion 230 may be formed in a V-groove shape, or the concave portion 230 may be formed to have a cross section in an arc shape.

According to the first modification example, a case in which the penetration holes 281 are formed along the direction orthogonal to the axis Q and formed to be opposite to each other with the axis Q as the center is described. However, it is not limited thereto, the penetration hole 281 only has to communicate the liquid accumulation portion 223 and the accommodation concave portion 220. It is suitable for at least one penetration holes 281 to be formed, and the penetration holes 281 may be formed in a plural number equal to two or more than two.

### (Second modification example)

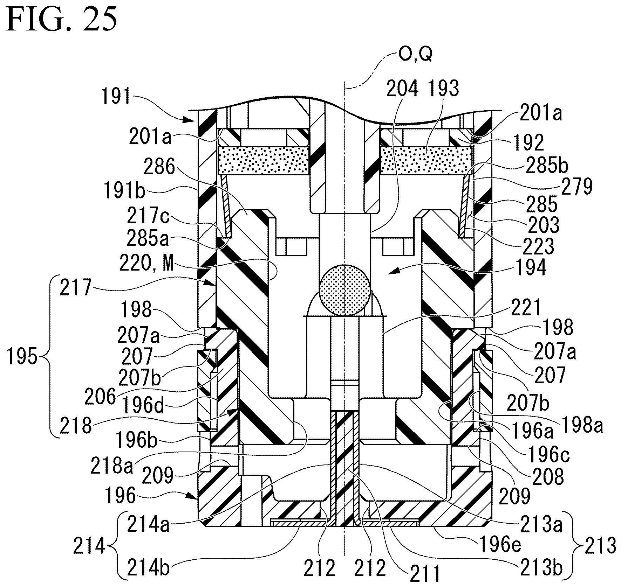

Next, a second modification example of the embodiment will be described with reference to Fig. 25.

Fig. 25 is an enlarged cross-sectional view in the axial direction showing a portion corresponding to the atomization container 195 of the cartridge 11 according to the second modification example. Fig. 25 corresponds to Fig. 13.

As shown in Fig. 25, the second modification example is different from the embodiment in that a shape of the atomization container 195 is different.

In the cylinder portion 217 of the atomization container 195, a portion for forming the liquid accumulation portion 223 is removed and a support member 285 different from the cylinder portion 217 is disposed in the removed portion. The removed surface of the cylinder portion 217 is referred to as a flat surface 217c orthogonal to the axis Q. A fitting convex portion 286 in a substantial cylindrical shape and protruding toward the mesh body 193 side is formed at a circumferential edge of the accommodation concave portion 220 on the flat surface 217c. The support member 285 is disposed on the flat surface 217c for positioning of the fitting convex portion 286.

The support member 285 is formed from a metal material. For example, it is desirable that the support member 285 is formed from stainless steel or the like having high rust-preventive performance. The support member 285 is formed in a substantial frusto-conical shape so as to be a substantial cylindrical shape and becoming wider as approaching from the flat surface 271c toward the mesh body 193 side when viewed from a direction orthogonal to the axis Q. An internal surface of a small-diameter portion 285a of the support member 285 fits to an external circumferential surface of the fitting convex portion 286 of the cylinder portion 217. Accordingly, the positioning of the support member 285 with respect to the cylinder portion 217 is performed.

An end portion of a large-diameter portion 285b of the support member 285 is in contact with the other surface 193c of the mesh body 193. An external diameter of the large-diameter portion 285b is set to be slightly smaller than the internal diameter of the circumferential wall 191b of the tank 191. Accordingly, a micro gap formed between the large-diameter portion 285b and the circumferential wall 191b of the tank 191 functions as the narrow portion 279.

The support member 285 is formed in the substantial frusto-conical shape as described above such that the gap between the support member 285 and the circumferential wall 191b of the tank 191 gradually becomes narrower from the small-diameter portion 285a toward the large-diameter portion 285b. The gap functions as the liquid accumulation portion 223.

In this manner, according to the cartridge 11 of the second modification example, the same effect may be achieved as the above-described embodiment. In addition, the cylinder portion 217 of the atomization container 195 is configured by being divided by the support member 285, and the liquid accumulation portion 223 is formed on the external circumferential surface of the support member 285. Accordingly, a moldability of the atomization container 195 may become easy. The support member 285 is formed from metal such that a mechanical strength of the support member 285 may be improved. The mesh body 193 may be definitely supported by an end portion of the large-diameter portion 285b of such support member 285.

### (Third modification example)

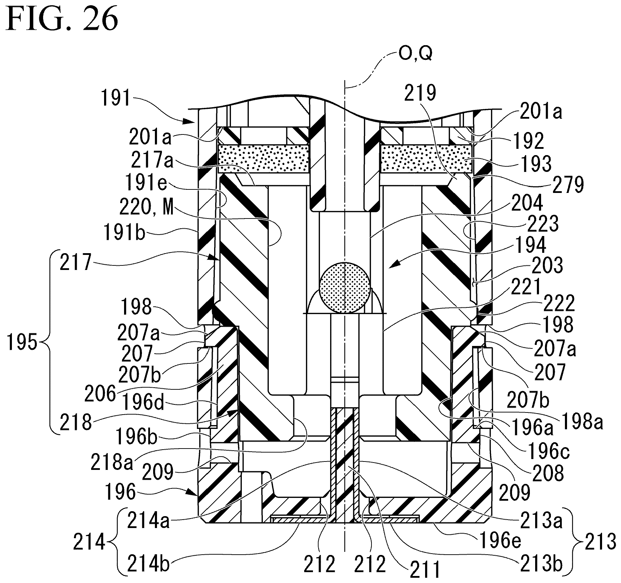

Next, a third modification example of the above-described embodiment will be described with reference to Fig. 26.

Fig. 26 is an enlarged cross-sectional view in the axial direction showing a portion corresponding to the atomization container 195 of the cartridge 11 according to the third modification example. Fig. 26 corresponds to Fig. 13.

As shown in Fig. 26, the second modification example and the above-described embodiment are different in that shapes of the atomization container 195 and the circumferential wall 191b of the tank 191 are different.

The external circumferential surface of the cylinder portion 217 of the atomization container 195 is not obliquely formed in an interval between the seal portion 222 and the tip end of the protrusion portion 219 and formed substantially paraellel to the axis Q.

On the other hand, in the circumferential wall 191b of the tank 191, the internal circumferential surface at the portion corresponding to the cylinder portion 217 is obliquely formed as an inclined surface 191e whose diameter gradually increases from the protrusion portion 219 of the cylinder portion 217 toward the seal portion 222. Accordingly, an interval between the circumferential surface of the cylinder portion 217 and the circumferential wall 191b of the tank 191 gradually becomes narrower toward the protrusion portion 219 of the cylinder portion 217.

Therefore, according to the above-described third modification example, the same effect may be achieved with the above-described embodiment.

In the third modification example, an external diameter of the seal portion 222 of the atomization container 195 becomes larger by the increase of the internal diameter of the circumferential wall 191b of the tank 191. Accordingly, in the state in which the atomization container 195 is accommodated in the tank 191, the seal portion 222 is compressed in the radial direction. Accordingly, it is possible to secure the seal performance of the seal portion 222 and prevent the slipping of the atomization container 195 from the tank 191 due to the friction resistance of the seal portion 222.

### (Fourth modification example)

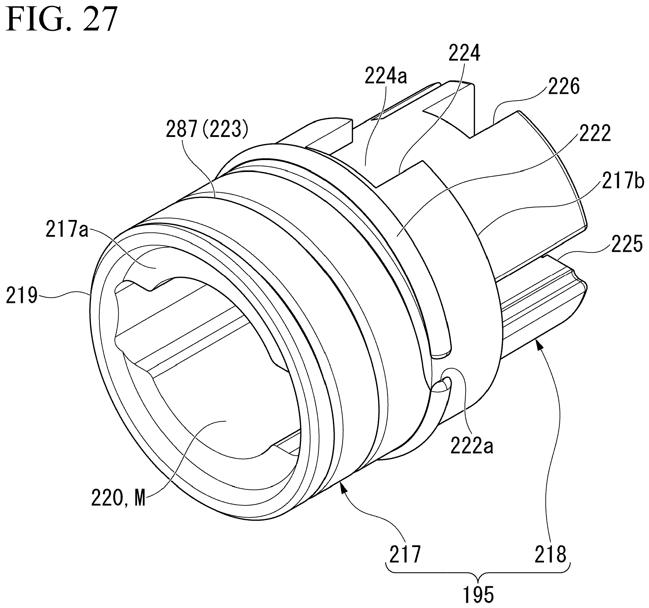

Next, a fourth modification example of the above-described embodiment will be described with reference to Fig. 27.

Fig. 27 is a perspective view of the atomization container 195 according to the fourth modification example viewed from the mesh body 193 side (second side in the axial direction). Fig. 27 corresponds to Fig. 17.

As shown in Fig. 27, the fourth modification example and the above-described embodiment are different in that the shape of the atomization container 195 is different.

The external circumferential surface of the cylinder portion 217 of the atomization container 195 is not obliquely formed in an interval between the seal portion 222 and the tip end of the protrusion portion 219 and formed substantially paraellel to the axis Q. A helix-shaped groove 287 is formed on the external circumferential surface of the cylinder portion 217. The groove 287 is formed between the seal portion 222 and an end portion of the protrusion portion 219. Accordingly, the groove 287 functions as the liquid accumulation portion 223.

An end portion of the groove 287 at the seal portion 222 side is communicated with the notch portion 222a formed in the seal portion 222. Accordingly, it is impossible to generate a pressure difference between the inside and outside the liquid accumulation portion 223.

In this manner, according to the cartridge 11 of the fourth modification example, the same effect with the above-described embodiment may be achieved. In addition, the groove 287 formed in the helix shape on the external circumferential surface of the cylinder portion 217 functions as the liquid accumulation portion 223. The groove 287 is formed in the helix shape such that it is difficult for the air to enter the groove 287. Accordingly, it may be easy for the liquid aerosol source stored in the groove 287 to flow back to the liquid storage room 202 of the tank 191.

### (Other modification example)

Preferred embodiments of the present invention have been described above, the present invention is not limited to the embodiments and modifications thereof. The present invention is not limited by the foregoing description and is limited only by the scope of the appended claims.

For example, according to the above-described embodiment, an example of the suction device 1 configured for the tobacco capsule 12 to be attachable to and detachable from is described as an example of an aerosol generation device for generating aerosol without combustion is described; however, the aerosol generation device is not limited to the configuration only. As another example of the aerosol generation device, a configuration without the tobacco capsule 12 such as an electrical tobacco may be provided. In this case, the aerosol source including a flavor is accommodated in the cartridge 11 and the aerosol including the flavor is generated by the aerosol generation device.

According to the above-described embodiment, a case in which the main body unit 10 is a divided configuration of the power unit 21, the retention unit 22, and the mouthpiece 23 is described; however, the main body unit 10 is not limited to the configuration only. For example, the power unit 21 and the retention unit 22 may be integrally formed, and the retention unit 22 and the mouthpiece 23 may be integrally formed.

According to the above-described embodiment, a configuration that the retention unit 22 is formed in a cylindrical shape to surround the circumference of the cartridge 11 is described; however, the retention unit 22 is not limited to the configuration only. The retention unit 22 only has to be a configuration capable of retaining the cartridge 11. In the present description, attachment and detachment of the cartridge 11 and the main body unit 10 (power unit 21) is not limited to the configuration of accommodating the cartridge 11 in the retention unit 22 and being retained by the mouthpiece 23, and the configuration of simply connecting or disconnecting the pin electrodes 49 with the connection electrodes 213b, 214b is included.

According to the above-described embodiment, a configuration that the power unit 21 and the retention unit 22 are formed in cylindrical shapes and disposed coaxially is described; however, the power unit 21 and the retention unit 22 are not limited only to this configuration. The power unit 21 and the retention unit 22 may be formed in different shapes.

According to the above-described embodiment, a configuration that the storage battery 33 and the substrate modules 34, 35 are carried on the storage battery holder 36 is described; however, the configuration is not limited thereto. The storage battery 33 and the substrate modules 34, 35 may be directed carried in the housing 31.

According to the above-described embodiment, a configuration of the button 78 (switch element 52) for outputting the start-up preparation signal is described; however, a configuration without the button 78 (a configuration for start-up by a detection by the pressure sensor 53) may be configured.

According to the above-described embodiment and each modification example, a case in which the liquid accumulation portion 223 is disposed in either of the external circumferential surface of the cylinder portion 217 or the circumferential wall 191b in the tank 191 is described. However, it is not limited only to the configuration. The concave portion may be formed in both of the external circumferential surface of the cylinder portion 217 or the circumferential wall 191b in the tank 191 to configure the liquid accumulation portion 223.

According to the above-described embodiment, a case in which the mesh body 193 is the porous member having liquid absorbency and formed from the cotton-type fibrous material, for example, is described. However, the mesh body 193 is not limited to the configuration. A plate-shaped member which does not have the liquid absorbency may be used instead of the mesh body 193. The inside of the tank 191 only has to be partitioned by the plate-shaped member into the liquid storage room 202 at the bottom 191c side and the opening room 203 at the opening portion 191a side. However, it is necessary to process the plate-shaped member so as to cause the liquid aerosol source stored in the liquid storage room 202 to be absorbed by the wick 4 through the plate-shaped member.

According to the above-described embodiment, a case in which the cylindrical portion 196b of the heater holder 196 is fitted to the internal circumferential surface of the circumferential wall 191b in the tank 191 is described. A case in which the engagement hole 198 is formed in the tank 191 and the engagement piece 206 is formed in the heater holder 196 so as to configure a means for engaging the tank 191 and the heater holder 196 with each other is described. However, it is not limited only to this configuration. The circumferential wall 191b of the tank 191 may be configured to fit to the internal circumferential surface of the cylindrical portion 196b of the heater holder 196. In this case, the engagement piece 206 is formed in the circumferential wall 191b of the tank 191 positioned at the internal side in the radial direction and the engagement hole 198 is formed in the cylindrical portion 196b of the heater holder 196 positioned at the external side in the radial direction.

The configuration only has to be engageable with the engagement piece 206, and may not configured as the engagement hole 198. In other words, a concave portion engageable with the engagement piece 206 may be configured instead of the engagement hole 198. According to such a configuration, a case in which the engagement claw 207 exposes to the outside through the engagement hole 198 will not occur. Accordingly, it is possible to configure the tank 191 and the heater holder 196 to be more difficult to disassemble.

According to the above-described embodiment, a case in which two engagement holes 198 are formed in the tank 191 and two engagement pieces 206 are formed in the heater holder 196 so as to fix the tank 191 and the heater holder 196 is described; however, it is not limited only to this configuration. Two or more than two engagement holes 198 and engagement pieces 206 may be formed in the tank 191 and the heater holder 196 respectively.

Part of the above-described embodiment or the whole embodiment may be disclosed in the following appendix and are not limited thereto.

### (Appendix 1)

An atomization unit comprises a tank in a bottomed cylindrical shape; and a holder fitted to an internal circumferential surface at an opening portion side of the tank, wherein two engagement pieces elastically deformable inwardly in a radial direction are disposed in the holder to protrude toward a bottom portion side of the tank, two engagement portions being engageable to the engagement pieces respectively are disposed in the tank, and the engagement pieces and the engagement portions are oppositely disposed with a radially central portion of the tank and the holder as a center.

### (Appendix 2)

The atomization unit comprises a container having elasticity for fitting to an internal circumferential surface of the holder.

### (Appendix 3)

In the atomization unit, the engagement pieces have engagement claws protruding from a tip end at the other side towards the outward in the radial direction, and the engagement portions have openings to which the engagement claws are insertable.

Additions, omissions, substitutions and other changes in the structure are possible within the scope of the appended claims. Each of the above-described modification examples may be suitably combined within the scope of the appended claims.

### [Industrial Applicability]

According to the above-described atomization unit, it is possible to cause the tank and the holder difficult to be intentionally disassembled. There is no necessity to form the groove portion over the whole circumference of the tank and the heater holder as in the past. Accordingly, it is possible to secure efficient mechanical strength of the tank and the heater holder.

## Claims

1. An atomization unit (11), comprising:
a tank (191) formed in a bottomed cylindrical shape in which liquid is stored; and
a holder (196) fitted to an opening portion of the tank (191),
wherein in a state in which the tank (191) and the holder (196) are fitted with each other,
at least an engagement piece (206) being elastically deformable towards inwardly in a radial direction is disposed in either of the tank (191) or the holder (196) to protrude from one of the tank (191) and the holder (196) positioned inwardly in the radial direction toward the other one of the tank (191) and the holder (196), and
an engagement portion being engageable with the engagement piece (206) is disposed in the other one of the tank (191) and the holder (196) positioned outwardly in the radial direction,
the atomization unit (11) is **characterized in that**:
the holder (196) is fitted to an internal circumferential surface of the tank (191),
an elastic applying member (195) is formed in an internal surface at an internal side in the radial direction of the engagement piece (206) so as to apply an elastic force outwardly in the radial direction to the engagement piece (206) in response to an elastic deformation of the engagement piece (206) inwardly in the radial direction, and
the elastic applying member (195) has a fitting portion (218) to be fitted to an internal circumferential surface of the holder (196) .

2. The atomization unit (11) according to Claim 1,
wherein the engagement piece (206) has an engagement claw (207) protruding from a tip end at the other one side towards outside in the radial direction, and
the engagement portion is an opening (198) in which the engagement claw (207) is insertable.

3. The atomization unit (11) according to Claim 2,
wherein the engagement claw (207) is formed to have a cross-sectional surface in a substantial triangle shape corresponding to a plane along an axial direction and a radial direction of the tank (191) and the holder (196),
the engagement claw (207) has an inclined surface (207a) which is inclined toward a base end side of the engagement piece (206) as towards outside in the radial direction from the tip end, and
a flat surface (207b) at the base end side is orthogonal to the axial direction.

4. The atomization unit (11) according to any one from Claim 1 to Claim 3,
wherein two of the engagement pieces (206) are included,
two of the engagement pieces (206) are oppositely disposed with a radially central portion of the tank (191) and the holder (196) as a center, and
two of the engagement portions are disposed corresponding to the two of the engagement pieces (206).

5. The atomization unit (11) according to any one from Claim 1 to Claim 4, wherein a guide concave portion (198a) configured to receive the engagement piece (206) to guide a fitting of the tank (191) and the holder (196) is formed in either of the tank (191) or the holder (196).

6. A non-combustion suction device (1), comprising:
the atomization unit (11) according to any one from Claim 1 to Claim 5;
a partition plate (193) with liquid absorbency and configured to partition the inside of the tank (191) into a liquid storage room (202) positioned at a bottom portion (191c) side of the tank (191) and an opening room (203) positioned at the opening portion (191a) side of the tank (191);
a wick (204) with the liquid absorbency and connected to the partition plate (193); and
a heater (194) having an electrical heating wire (205) for heating the wick (204),
wherein the holder (196), the wick (204), and the heater (194) are disposed at the opening room (203) side.

7. The non-combustion device (1) according to Claim 6, further comprising:
a container retention cylinder (120) configured to accommodate the atomization unit (11);
a power portion (21) connected to an end portion of the container retention cylinder (120) at the opposite side of the bottom portion (191c) of the tank (191); and
the holder (196) in a bottomed cylindrical shape,
wherein a bottom portion (196e) of the holder (196) is disposed to face the opposite side of the bottom portion (191c) of the tank (191), and
an electrode (213, 214) is formed in the bottom portion (196e) of the holder (196) to be connected to the electrical heating wire (205) and be able to come in contact with a pin electrode (49) of the power portion (21).

## Patentansprüche

1. Verneblereinheit (11), umfassend:
einen Behälter (191), der in einer mit einem Boden versehenen zylindrischen Form geformt ist und in dem Flüssigkeit gespeichert ist; und
einen Halter (196), der an einem Öffnungsabschnitt des Behälters (191) angebracht ist,
wobei in einem Zustand, in dem der Behälter (191) und der Halter (196) aneinander angebracht sind,
mindestens ein Eingriffsstück (206), das in einer radialen Richtung nach innen elastisch verformbar ist, entweder in dem Behälter (191) oder in dem Halter (196) angeordnet ist, um von einem von dem Behälter (191) und dem Halter (196), der in der radialen Richtung nach innen positioniert ist, zu dem anderen von dem Behälter (191) und dem Halter (196) vorzustehen, und
ein Eingriffsabschnitt, der mit dem Eingriffsstück (206) in Eingriff gebracht werden kann, in dem anderen von dem Behälter (191) und dem Halter (196) angeordnet ist, der in der radialen Richtung nach außen positioniert ist,
wobei die Verneblereinheit (11) **dadurch gekennzeichnet ist, dass**:
der Halter (196) an einer inneren Umfangsfläche des Behälters (191) angebracht ist,
ein elastisches Aufbringungselement (195) in einer Innenfläche an einer Innenseite in der radialen Richtung des Eingriffsstücks (206) ausgebildet ist, um damit als Reaktion auf eine elastische Verformung des Eingriffsstücks (206) nach innen in der radialen Richtung eine elastische Kraft nach außen in der radialen Richtung auf das Eingriffsstück (206) aufzubringen, und
das elastische Aufbringungselement (195) einen Anbringungsabschnitt (218) aufweist, um an einer inneren Umfangsfläche des Halters (196) angebracht zu werden.

2. Verneblereinheit (11) nach Anspruch 1,
wobei das Eingriffsstück (206) eine Eingriffsklaue (207) aufweist, die von einem Spitzenende an der anderen einen Seite in der radialen Richtung nach außen vorsteht, und
der Eingriffsabschnitt eine Öffnung (198) ist, in die die Eingriffsklaue (207) einführbar ist.

3. Verneblereinheit (11) nach Anspruch 2,
wobei die Eingriffsklaue (207) ausgebildet ist, um eine Querschnittsfläche in einer im Wesentlichen dreieckigen Form aufzuweisen, die einer Ebene entlang einer axialen Richtung und einer radialen Richtung des Behälters (191) und des Halters (196) entspricht,
die Eingriffsklaue (207) eine geneigte Oberfläche (207a) aufweist, die in Richtung einer Basisendseite des Eingriffsstücks (206) nach außen in der radialen Richtung von dem Spitzenende geneigt ist, und
eine ebene Oberfläche (207b) an der Basisendseite orthogonal zu der axialen Richtung ist.

4. Verneblereinheit (11) nach einem von Anspruch 1 bis Anspruch 3,
wobei zwei der Eingriffsstücke (206) beinhaltet sind,
zwei der Eingriffsstücke (206) einander gegenüberliegend angeordnet sind, wobei ein radial zentraler Abschnitt des Behälters (191) und des Halters (196) als ein Zentrum dient, und
zwei der Eingriffsabschnitte entsprechend den beiden Eingriffsstücken (206) angeordnet sind.

5. Verneblereinheit (11) nach einem von Anspruch 1 bis Anspruch 4, wobei entweder in dem Behälter (191) oder in dem Halter (196) ein konkaver Führungsabschnitt (198a) ausgebildet ist, der zum Aufnehmen des Eingriffsstücks (206) konfiguriert ist, um eine Anbringung des Behälters (191) und des Halters (196) zu führen.

6. Verbrennungsfreie Saugvorrichtung (1), umfassend:
die Verneblereinheit (11) nach einem von Anspruch 1 bis Anspruch 5;
eine Unterteilungsplatte (193) mit Flüssigkeitsabsorptionsvermögen und die konfiguriert ist, um den Innenraums des Behälters (191) in einen Flüssigkeitsspeicherraum (202), der an einer Seite des Bodenabschnitts (191c) des Behälters (191) positioniert ist, und einen Öffnungsraum (203), der an der Seite des Öffnungsabschnitts (191a) des Behälters (191) positioniert ist, zu unterteilen;
einen Docht (204) mit der Flüssigkeitsabsorptionsfähigkeit und der mit der Unterteilungsplatte (193) verbunden ist; und
eine Heizvorrichtung (194), die einen elektrischen Heizdraht (205) zum Beheizen des Dochts (204) aufweist,
wobei der Halter (196), der Docht (204) und die Heizvorrichtung (194) auf der Seite des Öffnungsraums (203) angeordnet sind.

7. Verbrennungsfreie Vorrichtung (1) nach Anspruch 6, weiter umfassend:
einen Behälteraufbewahrungszylinder (120), der zum Aufnehmen der Verneblereinheit (11) konfiguriert ist;
einen Leistungsabschnitt (21), der mit einem Endabschnitt des Behälteraufbewahrungszylinders (120) auf der dem Bodenabschnitt (191c) des Behälters (191) gegenüberliegenden Seite verbunden ist; und
den Halter (196) in einer mit einem Boden versehenen zylindrischen Form,
wobei ein Bodenabschnitt (196e) des Halters (196) angeordnet ist, um der gegenüberliegenden Seite des Bodenabschnitts (191c) des Behälters (191) zugewandt zu sein, und
eine Elektrode (213, 214) in dem Bodenabschnitt (196e) des Halters (196) ausgebildet ist, um mit dem elektrischen Heizdraht (205) verbunden zu sein und in der Lage zu sein, mit einer Stiftelektrode (49) des Leistungsabschnitts (21) in Kontakt zu kommen.

## Revendications

1. Unité d'atomisation (11), comprenant :
un réservoir (191) de forme cylindrique pourvue d'un fond dans lequel un liquide est stocké ; et
un support (196) monté sur une partie d'ouverture du réservoir (191),
dans laquelle dans un état dans lequel le réservoir (191) et le support (196) sont emboîtés l'un dans l'autre,
au moins une pièce d'engagement (206) qui est déformable élastiquement vers l'intérieur dans une direction radiale est disposée soit dans le réservoir (191) soit dans le support (196) pour faire saillie depuis l'un du réservoir (191) et du support (196) positionné vers l'intérieur dans la direction radiale vers l'autre du réservoir (191) et du support (196), et
une partie d'engagement pouvant être engagée avec la pièce d'engagement (206) est disposée dans l'autre du réservoir (191) et du support (196) positionné vers l'extérieur dans la direction radiale,
l'unité d'atomisation (11) est **caractérisée en ce que** :
le support (196) est monté sur une surface circonférentielle interne du réservoir (191),
un élément d'application élastique (195) est formé dans une surface interne au niveau d'un côté interne dans la direction radiale de la pièce d'engagement (206) de manière à appliquer une force élastique vers l'extérieur dans la direction radiale à la pièce d'engagement (206) en réponse à une déformation élastique de la pièce d'engagement (206) vers l'intérieur dans la direction radiale, et
l'élément d'application élastique (195) présente une partie de montage (218) à monter sur une surface circonférentielle interne du support (196).

2. Unité d'atomisation (11) selon la revendication 1,
dans laquelle la pièce d'engagement (206) présente une griffe d'engagement (207) faisant saillie à partir d'une extrémité de pointe au niveau de l'autre côté vers l'extérieur dans la direction radiale, et
la partie d'engagement est une ouverture (198) dans laquelle la griffe d'engagement (207) peut être insérée.

3. Unité d'atomisation (11) selon la revendication 2,
dans laquelle la griffe d'engagement (207) est formée pour présenter une surface en coupe transversale en forme sensiblement de triangle correspondant à un plan le long d'une direction axiale et d'une direction radiale du réservoir (191) et du support (196),
la griffe d'engagement (207) présente une surface inclinée (207a) qui est inclinée vers un côté d'extrémité de base de la pièce d'engagement (206) comme vers l'extérieur dans la direction radiale à partir de l'extrémité de pointe, et
une surface plane (207b) au niveau du côté d'extrémité de base est orthogonale à la direction axiale.

4. Unité d'atomisation (11) selon l'une quelconque des revendications 1 à 3,
dans laquelle deux des pièces d'engagement (206) sont incluses,
deux des pièces d'engagement (206) sont disposées de manière opposée avec une partie radialement centrale du réservoir (191) et du support (196) comme centre, et
deux des parties d'engagement sont disposées en correspondance avec les deux pièces d'engagement (206).

5. Unité d'atomisation (11) selon l'une quelconque des revendications 1 à 4, dans laquelle une partie concave de guidage (198a) configurée pour recevoir la pièce d'engagement (206) pour guider un montage du réservoir (191) et du support (196) est formée dans le réservoir (191) ou dans le support (196).

6. Dispositif d'aspiration sans combustion (1), comprenant :
l'unité d'atomisation (11) selon l'une quelconque des revendications 1 à 5 ;
une plaque de séparation (193) avec une capacité d'absorption des liquides et configurée pour diviser l'intérieur du réservoir (191) en une chambre de stockage de liquide (202) positionnée au niveau d'un côté d'une partie inférieure (191c) du réservoir (191) et une chambre d'ouverture (203) positionnée au niveau de la partie d'ouverture (191a) du réservoir (191) ;
une mèche (204) avec une capacité d'absorption des liquides et reliée à la plaque de séparation (193) ; et
un dispositif de chauffage (194) présentant un fil chauffant électrique (205) pour chauffer la mèche (204),
dans lequel le support (196), la mèche (204), et le dispositif de chauffage (194) sont disposés du côté de la chambre d'ouverture (203).

7. Dispositif sans combustion (1) selon la revendication 6, comprenant en outre :
un cylindre de retenue de récipient (120) configuré pour loger l'unité d'atomisation (11) ;
une partie d'alimentation (21) reliée à une partie d'extrémité du cylindre de retenue de récipient (120) au niveau du côté opposé de la partie inférieure (191c) du réservoir (191) ; et
le support (196) dans une forme cylindrique pourvue d'un fond,
dans lequel une partie inférieure (196e) du support (196) est disposée de manière à faire face au côté opposé de la partie inférieure (191c) du réservoir (191), et
une électrode (213, 214) est formée dans la partie inférieure (196e) du support (196) pour être connectée au fil chauffant électrique (205) et pouvoir entrer en contact avec une électrode à broche (49) de la partie d'alimentation (21).
